# EUROPÄISCHE PATENTSCHRIFT

(11) **EP 0 584 611 B1**
(45) Veröffentlichungstag und Bekanntmachung des Hinweises auf die Patenterteilung: **17.01.1996**
(21) Anmeldenummer: 93112613.0
(22) Anmeldetag: 06.08.1993
(51) Int. Cl.: C07D 471/04

(54) **Heteroaromatisch kondensierte Hydroxypyridoncarbonsäureamide, deren Herstellung und Verwendung**
Heteroaromatically condensed hydroxypyridone carboxamides, their preparation and use
Hydroxypyridone carboxamides hétéroaromatiquement condensés, leur préparation et leur utilisation

(30) Priorität: 21.08.1992 DE 4227747
(43) Veröffentlichungstag der Anmeldung: 02.03.1994
(73) Patentinhaber: BASF Aktiengesellschaft, D-67063 Ludwigshafen (DE)
(72) Erfinder: Nuebling, Christoph, Dr., D-6733 Hassloch (DE); von Deyn, Wolfgang, Dr., D-6730 Neustadt (DE); Theobald, Hans, Dr., D-6703 Limburgerhof (DE); Westphalen, Karl-Otto, Dr., D-6720 Speyer (DE); Kardorff, Uwe, Dr., D-6800 Mannheim (DE); Walter, Helmut, Dr., D-6719 Obrigheim (DE); Kappe, Thomas, Prof.Dr., D-8010 Graz (AT); Gerber, MAtthias, Dr., D-6703 Limburgerhof (DE)

(56) Entgegenhaltungen:
- EP-A- 0 292 990
- EP-A- 0 544 151
- EP-A- 0 544 152
- DATABASE WPI Week 6800, Derwent Publications Ltd., London, GB; AN 66-34494F
- DATABASE WPI Week 9212, Derwent Publications Ltd., London, GB; AN 92-092970
- CHEMICAL ABSTRACTS, vol. 102, no. 19, 13. Mai 1985, Columbus, Ohio, US; abstract no. 166772c,
- JOURNAL OF THE CHEMICAL SOCIETY, PERKIN TRANSACTIONS 1 1990, LETCHWORTH GB Seiten 2943 - 2950 Y.S. SANGHVI ET AL.

## Beschreibung

Die vorliegende Erfindung betrifft heteroaromatisch kondensierte Hydroxypyridoncarbonsäureamide der Struktur I 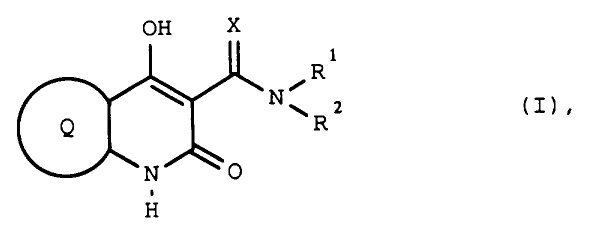 in der die Substituenten und der Ring Q folgende Bedeutung haben:
- R¹: Wasserstoff, Hydroxyl, C₁-C₁₂-Alkyl, C₃-C₁₂-Alkenyl, C₃-C₁₂-Alkinyl, C₃-C₈-Cycloalkyl, C₁-C₁₂-Alkoxy, wobei die organischen Reste substituiert sein können;
- R: Wasserstoff, C₁-C₁₂-Alkyl, C₃-C₁₂-Alkenyl, C₃-C₁₂-Alkinyl, C₃-C₈-Cycloalkyl, C₃-C₈-Alkenyloxy, Di-C₁-C₄-Alkylamino, wobei die organischen Reste ggf. substituiert sind;
oder
- R¹, R: gemeinsam eine Alkylenkette mit 2 - 6 Gliedern bedeuten, welche durch Sauerstoff, Schwefel oder N-Methyl unterbrochen sein kann;
- X: Sauerstoff oder Schwefel;
- Q: ein fünf- oder sechsgliedriger heteroaromatischer Ring enthaltend 1 - 3 Stickstoffatome und/oder ein Sauerstoff- oder Schwefelatom als Heteroatome, welcher ein- bis dreimal substituiert sein kann
und deren umweltverträglichen Salze.

Insbesondere betrifft die Erfindung heteroaromatisch kondensierte Hydroxypyridoncarbonsäureamide der allgemeinen Formel I 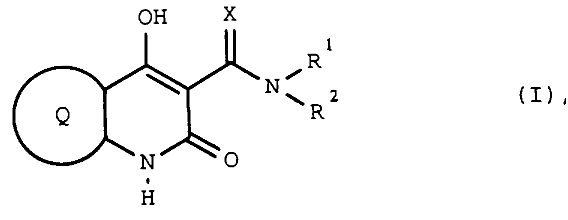 in der die Substituenten und der Ring Q folgende Bedeutung haben:
- R¹: Wasserstoff, Hydroxyl, C₁-C₁₂-Alkyl, C₃-C₁₂-Alkenyl, C₃-C₁₂-Alkinyl, C₃-C₈-Cycloalkyl, C₁-C₁₂-Alkoxy, wobei diese Gruppen folgende Reste tragen können: Halogen, Cyano, C₃-C₈-Cycloalkyl, C₁-C₄-Alkoxy, C₁-C₄-Alkylthio, C₁-C₄-Halogenalkylthio, C₁-C₄-Halogenalkoxy, Di-C₁-C₄-Alkylamino, Phenyl, Phenoxy, Phenylthio, wobei die Phenylreste ihrerseits eine bis drei der folgenden Gruppen tragen können: Halogen, C₁-C₄-Alkyl, Cyano, Nitro, C₁-C₄-Alkoxy, C₁-C₄-Halogenalkyl, C₁-C₄-Halogenalkoxy
- R: Wasserstoff, C₁-C₁₂-Alkyl, C₃-C₁₂-Alkenyl, C₃-C₁₂-Alkinyl, C₃-C₈-Cycloalkyl, C₃-C₈-Alkenyloxy, Di-C₁-C₄-Alkylamino, wobei diese Gruppen folgende Reste tragen können: Halogen, Cyano, C₃-C₈-Cycloalkyl, C₁-C₄-Alkoxy, C₁-C₄-Alkylthio, C₁-C₄-Halogenalkoxy, C₁-C₄-Halogenalkylthio, Phenyl, Phenoxy, Phenylthio, wobei die Phenylreste ihrerseits eine bis drei der folgenden Gruppen tragen können: Halogen C₁-C₄-Alkyl, C₁-C₄-Alkoxy, Cyano, Nitro
oder
- R¹, R: gemeinsam eine Alkylenkette mit 2 - 6 Gliedern bedeuten, welche durch Sauerstoff, Schwefel oder N-Methyl unterbrochen sein kann
- X: Sauerstoff oder Schwefel
- Q: ein fünf- oder sechsgliedriger heteroaromatischer Ring enthaltend 1 - 3 Stickstoffatome und/oder ein Sauerstoff- oder Schwefelatom als Heteroatome, welcher ein bis drei der folgenden Reste tragen kann: Halogen, Nitro, Cyano, C₁-C₄-Alkyl, C₁-C₄-Alkoxy, C₁-C₄-Alkylthio, C₁-C₄-Alkoxy-C₁-C₄-alkyl, C₃-C₈-Cycloalkyl, C₂-C₄-Alkenyl, Phenyl, Phenyl-C₁-C₄-alkyl, wobei die Phenylreste ihrerseits ein bis drei der folgenden Gruppen tragen können: Halogen, C₁-C₄-Alkyl, C₁-C₄-Alkoxy, C₁-C₄-Alkylthio, C₁-C₄-Halogenalkyl, C₁-C₄-Halogenalkoxy
und deren umweltverträgliche Salze.

Außerdem betrifft die Erfindung die Herstellung der Verbindungen I und ihre Verwendung als Herbizide.

Es ist bekannt, daß 1-Phenyl-substituierte 4-Hydroxy-1,8-naphthyrid-2-on-3-carbonsäureamide entzündungshemmende Eigenschaften aufweisen (J. Med. Chem. 35 (1992) 1130 und EP-A 452873). Ein Hinweis auf eine herbizide Wirkung ist dem Stand der Technik jedoch nicht zu entnehmen. In den älteren deutschen Anmeldungen P 41 38 819.4 und P 41 38 820.8 werden nicht kondensierte Hydroxypyridoncarbonsäureamide und Hydroxychinolincarbonsäureamide beschrieben.

In der europäischen Anmeldung EP-A 292 990 werden 4-Halopyridin-3-carboxamide mit herbizider Wirkung und in der japanischen Anmeldung JP-A-43023948 2,4-Dihydroxychinolin-3-carbonsäureamide mit antibakterieller Wirkung beschrieben.

Der Erfindung lag nun die Aufgabe zugrunde, neue Hydroxypyridoncarbonsäureamide mit guter herbizider Wirkung sowie Verfahren zu deren Herstellung bereitzustellen.

Demgemäß wurden die eingangs definierten heteroaromatisch kondensierten Hydroxypyridoncarbonsäureamide der Formel I, Verfahren zu ihrer Herstellung sowie herbizide Mittel, die die Verbindungen I enthalten, gefunden.

Die Verbindungen der Formel I können in folgenden tautomeren Formen vorliegen, die ebenfalls von der Erfindung umfaßt werden:

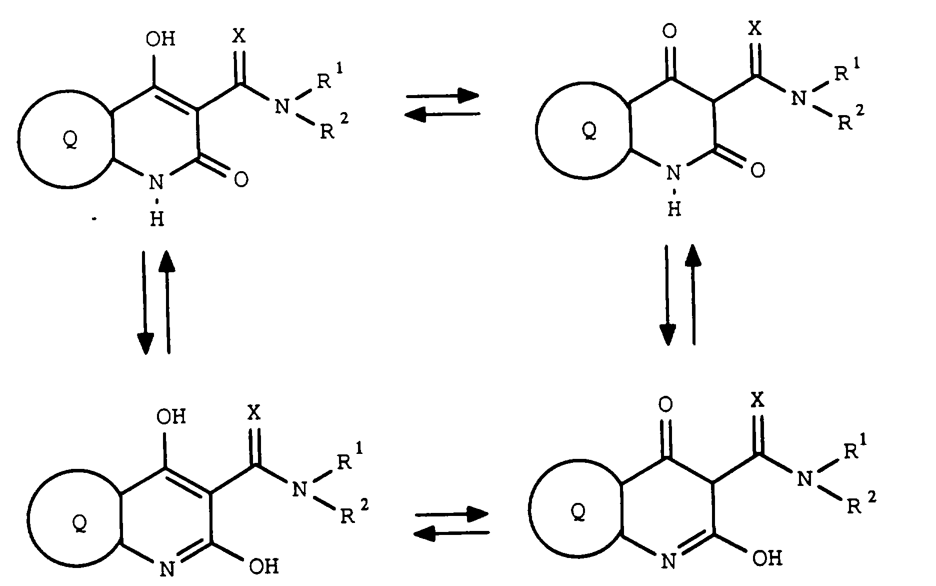

Die Herstellung der Verbindungen I gelingt in an sich bekannter Weise durch Umsetzung von Verbindungen der Formel II 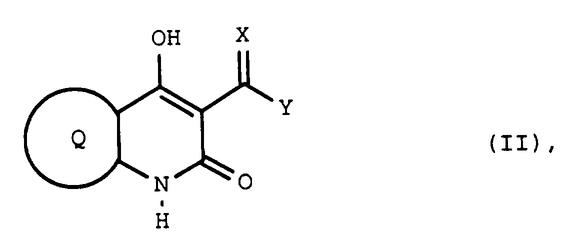 in der X und Q die oben angegebenen Bedeutungen haben und Y Hydroxyl, Halogen oder eine übliche nukleofuge Abgangsgruppe bedeutet, mit einem Amin der Formel III 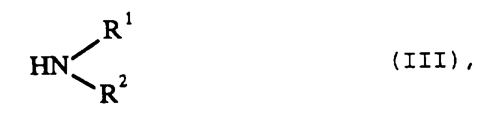 in der R¹ und R die oben angegebenen Bedeutungen haben.

Die Umsetzung wird üblicherweise bei Temperaturen von 20°C bis 250°C, vorzugsweise 100°C bis 180°C in einem inerten organischen Lösungsmittel durchgeführt.

Als Lösungs- oder Verdünnungsmittel eignen sich beispielsweise aliphatische, alicyclische und aromatische Kohlenwasserstoffe wie Pentan, Hexan, Cyclohexan, Benzol, Toluol, Xylole; Ether wie Diethyl-, Di-n-butylether, Methyl-tert.-butylether, Dimethoxyethan, Ethylenglykoldimethylether, Tetrahydrofuran und Dioxan; Alkohole wie Methanol, Ethanol, Propanol und Butanol, aprotische dipolare Lösungsmittel wie Dimethylformamid, Dimethylsulfoxid oder Pyridin.

Es können auch Gemische dieser Stoffe als Lösungs- und Verdünnungsmittel verwendet werden.

Die Ausgangsstoffe werden üblicherweise in stöchiometrischen Mengen miteinander umgesetzt. Es kann, beispielsweise zur Steigerung der Ausbeute, vorteilhaft sein, einen der Ausgangsstoffe, vorzugsweise das Amin, in einem Überschuß von 0,1 bis 10 mol-Äquivalenten zu verwenden.

Die Umsetzung wird im allgemeinen bei Atmosphärendruck ausgeführt. Es kann jedoch, je nach Art des verwendeten Amins oder Lösungsmittels, vorteilhaft sein, die Umsetzung unter erhöhtem Druck, insbesondere unter autogen erhöhtem Druck in einem Autoklaven durchzuführen.

Die Art der im Hydroxypyridoncarbonsaurederivat II verwendeten nukleofugen Abgangsgruppe Y spielt für die Amidierungsreaktion keine große Rolle, so daß man vorzugsweise einfach und billig zugängliche Derivate II einsetzt. Neben Y = OH und Halogen, insbesondere Chlor oder Brom kann Y für eine Gruppe OR oder SR stehen, wobei R einen aliphatischen, cycloaliphatischen oder araliphatischen Rest bedeuten kann. Vorteilhaft werden die freien Carbonsäuren (Y = OH), die Säurehalogenide, insbesondere Säurechloride (Y = Cl) oder die Ester- bzw. Thioester mit niedermolekularen Alkylresten (Y = OR, SR mit R = C₁-C₆-Alkyl, z.B. Methyl oder Ethyl) umgesetzt.

Weiterhin erhält man die Verbindungen der Formel I in einer Stufe aus den entsprechenden Sauren II (X = O, Y = OH) durch Umsetzung mit einem Amin III in Gegenwart eines wasserentziehenden Mittels wie z.B. Dicyclohexylcarbodiimid bei Temperaturen von 0- 100°C, 5 bevorzugt 0 - 30°C in einem inerten Lösungsmittel wie Dichlormethan, Tetrahydrofuran, Toluol oder Ethylacetat. Solche Umsetzungen sind z.B. in WO 90/15052 beschrieben.

Die Säuren II (X = O, Y = OH) sind aus den Estern II (X = O, Y = O-Alkyl) durch Verseifung mit Bariumhydroxid in an sich bekannter Weise (J. Chem. Soc. 1963, 491) erhältlich.

Die für die Umsetzungen benötigten Amine III sind bekannt, kommerziell erhältlich oder lassen sich nach allgemein bekannten chemischen Verfahren herstellen.

Die Ausgangsverbindungen der Formel II sind entweder bekannt oder lassen sich nach allgemein bekannten Verfahren darstellen. Naphthyridincarbonsäureester der Formel II (Q = Pyridin) sind z.B. beschrieben in: J. Med. Chem. 30 (1987) 2270; J. Prakt. Chem. 333 (1991) 637; US-A-4 215 123; Pol. J. Chem. 52 (1978) 2369 und Rocz. Chem. 48 (1974) 1815 (CA 82, 111959 g). Pyridopyrimidine der Formel II (Q = Pyrimidin) sind z.B. beschrieben in: Chem. Ber. 96 (1963) 1868 und JP-A 59210-093 (CA 102, 166772 c), N-alkylierte Derivate z.B. in US-A-4 215 216. Thienopyridine der Formel II (Q = Thiophen) sind z.B. beschrieben in: J. Chem. Res. (S) 1989, 196; J. Chem. Res. (S) 1985, 214 und J. Chem. Res. (S) 1980, 6. Pyrazolopyridine der Formel II (Q = Pyrazol) sind z.B. beschrieben in: J. Heterocycl. Chem. 15 (1978) 319 und J. Chem. Soc., Perkin Trans. I, 1990, 2943. Isoxazolopyridine der Formel II (Q = Isoxazol) sind z.B. beschrieben in: J. Chem. Soc., Perkin Trans. I, 1982, 2391 und J. Heterocycl. Chem. 14 (1977) 435. Pyrrolopyridine der Formel II (Q = Pyrrol) können analog der in BE-A 826 926 angegebenen Methode dargestellt werden. Die hier nicht explizit genannten Ausgangsverbindungen der Formel II lassen sich analog der zitierten Herstellungsverfahren durch Kondensation eines heterocyclischen 1-Amino-2-carbonsäureesters mit einem Malonsäurederivat darstellen.

Im Hinblick auf die bestimnungsgemäße Verwendung der heteroaromatisch kondensierten Hydroxypyridoncarbonsäureamide der allgemeinen Formel I kommen als Substituenten folgende Reste in Betracht:
- R¹: Wasserstoff,
unverzweigtes oder verzweigtes C₁-C₁₂-Alkyl, wie Methyl, Ethyl, Propyl, 1-Methylethyl, Butyl, 1-Methylpropyl, 2-Methylpropyl, 1,1-Dimethylethyl, Pentyl, 1-Methylbutyl, 2-Methylbutyl, 3-Methylbutyl, 1,1-Dimethylpropyl, 1,2-Dimethylpropyl, 2,2-Dimethylpropyl, 1-Ethylpropyl, Hexyl, 1-Methylpentyl, 2-Methylpentyl, 3-Methylpentyl, 4-Methylpentyl, 1,1-Dimethylbutyl, 1,2-Dimethybutyl, 1,3-Dimethylbutyl, 2,2-Dimethylbutyl, 2,3-Dimethylbutyl, 3,3,-Dimethylbutyl, 1-Ethylbutyl, 1,1,2-Trimethylpropyl, 1,2,2-Trimethylpropyl, 1-Ethyl-1-methylpropyl, 1-Ethyl-2-methylpropyl, Heptyl, 1-Methylheptyl, Octyl, Nonyl, Decyl, Undecyl und Dodecyl,
insbesondere C₁-C₆-Alkyl wie Methyl, Ethyl, 1-Methylethyl, 1-Methylpropyl, 2-Methylpropyl, 1,1-Dimethylethyl und 1,1-Dimethylpropyl;
unverzweigtes oder verzweigtes C₃-C₁₂-Alkenyl wie 2-Propenyl, 2-Butenyl, 3-Butenyl, 1-Methyl-2-propenyl, 2-Methyl-2-propenyl, 2-Pentenyl, 3-Pentenyl, 4-Pentenyl, 3-Methyl-2-butenyl, 1-Methyl-2-butenyl, 2-Methyl-2-butenyl, 1-Methyl-3-butenyl, 2-Methyl-4-butenyl, 3-Methyl-3-butenyl, 1,1-Dimethyl-2-propenyl, 1,2-Dimethyl-2-propenyl, 1-Ethyl-2-propenyl, 2-Hexenyl, 3-Hexenyl, 4-Hexenyl, 5-Hexenyl, 1-Methyl-2-pentenyl, 2-Methyl-2-pentenyl, 3-Methyl-2-pentenyl, 4-Methyl-2-pentenyl, 1-Methyl-3-pentenyl, 2-Methyl-2-pentenyl, 3-Methyl-3-pentenyl, 4-Methyl-3-pentenyl, 1-Methyl-4-pentenyl, 2-Methyl-4-pentenyl, 3-Methyl-4-pentenyl, 4-Methyl-4-pentenyl, 1,1-Dimethyl-2-butenyl, 1,1-Dimethyl-3-butenyl, 1,2-Dimethyl-2-butenyl, 1,2-Dimethyl-3-butenyl, 1,3-Dimethyl-2-butenyl, 1,3-Dimethyl-3-butenyl, 2,2-Dimethyl-3-butenyl, 2,3-Dimethyl-2-butenyl, 2,3-Dimethyl-3-butenyl, 1-Ethyl-2-butenyl, 1-Ethyl-3-butenyl, 2-Ethyl-2-butenyl, 2-Ethyl-3-butenyl, 1,1,2-Trimethyl-2-propenyl, 1-Ethyl-1-methyl-2-propenyl, Ethyl-2-methyl-2-propenyl und 10-Undecenyl,
insbesondere C₃-C₆-Alkenyl wie 2-Propenyl, 2-Butenyl, 3-Butenyl, 1-methyl-2-propenyl, 2-Methyl-2-propenyl 1-Methyl-2-butenyl und 1,1-Dimethyl-2-propenyl;
C₃-C₁₂-Alkinyl, insbesondere C₃-C₆-Alkinyl wie Propargyl, 2-Butinyl, 3-Butinyl, 2-Pentinyl, 3-Pentinyl, 4-Pentinyl, 1-Methyl-3-butinyl, 2-Methyl-3-butinyl, 1-Methyl-2-butinyl, 1,1-Dimethyl-2-propinyl, 1-Ethyl-2-propinyl, 2-Hexinyl, 3-Hexinyl, 4-Hexinyl, 5-Hexinyl, 1-Methyl-2-pentinyl, 1-Methyl-3-pentinyl, 1-Methyl-4-pentinyl, 3-Methyl-4-pentinyl, 4-Methyl-2-pentinyl, 1,1-Dimethyl-2-butinyl, 1,1-Dimethyl-3-butinyl, 1,2-Dimethyl-3-butinyl, 2,2-Dimethyl-3-butinyl, 1-Ethyl-2-butinyl, 1-Ethyl-3-butinyl, 2-Ethyl-3-butinyl und 1-Ethyl-1-methyl-2-propinyl; C₃-C₈-Cycloalkyl wie Cyclopropyl, Cyclobutyl, Cyclopentyl, Cyclohexyl, Cycloheptyl, Cyclooctyl, insbesondere Cyclopropyl und Cyclohexyl;
C₁-C₁₂-Alkoxy, insbesondere C₁-C₆-Alkoxy wie Methyloxy, Ethyloxy, Propyloxy, 1-Methylethyloxy, Butyloxy, 1-Methylpropyloxy, 2-Methylpropyloxy, 1,1-Dimethylethyloxy, Pentyloxy, 1-Methylbutyloxy, 2-Methylbutyloxy, 3-Methylbutyloxy, 1,1-Dimethylpropyloxy, 1,2-Dimethylpropyloxy, 2,2-Dimethylpropyloxy, 1-Ethylpropyloxy, Hexyloxy, 1-Methylpentyloxy, 2-Methylpentyloxy, 3-Methylpentyloxy, 4-Methylpentyloxy, 1,1-Dimethylbutyloxy, 1,2-Dimethylbutyloxy, 1,3-Dimethylbutyloxy, 2,2-Dimethylbutyloxy, 2,3-Dimethylbutyloxy, 3,3-Dimethylbutyloxy, 1-Ethylbutyloxy, 2-Ethylbutyloxy, 1,1,2-Trimethylpropyloxy, 1,2,2-Trimethylpropyloxy, 1-Ethyl-1-methylpropyloxy und 1-Ethyl-2-methylpropyloxy,
wobei die vorstehend genannten organischen Gruppen ein bis fünf Halogenatome wie Fluor, Chlor, Brom und Iod, vorzugsweise Fluor, Chlor und Brom, insbesondere Fluor und Chlor,
und/oder einen bis drei der folgenden Reste tragen können:
Cyano;
C₃-C₈-Cycloalkyl, wie Cyclopropyl, Cyclobutyl, Cyclopentyl, Cyclohexyl, Cycloheptyl, Cyclooctyl, insbesondere Cyclopropyl und Cyclohexyl;
C₁-C₄-Alkoxy wie Methoxy, Ethoxy, n-Propoxy, 2-Methylethoxy, n-Butoxy, 1-Methylpropoxy, 2-Methylpropoxy und 1,1-Dimethylethoxy, insbesondere Methoxy und Ethoxy;
C₁-C₄-Halogenalkoxy wie Chlormethoxy, Dichlormethoxy, Trichlormethoxy, Fluormethoxy, Difluormethoxy, Trifluormethoxy, Chlordifluormethoxy, Dichlorfluormethoxy, 1-Fluorethoxy, 2-Fluorethoxy, 2,2-Difluorethoxy, 1,1,2,2-Tetrafluorethoxy, 2,2,2-Trifluorethoxy, 2-Chlor-1,1,2-trifluorethoxy und Pentafluorethoxy, insbesondere Trifluormethoxy, 2,2,2-Trifluorethyloxy und 2-Chlor-2,2-difluorethoxy;
C₁-C₄-Alkylthio wie Methylthio, Ethylthio, n-Propylthio, 1-Methylethylthio, n-Butylthio, 1-Methylpropylthio, 2-Methylpropylthio und 1,1-Dimethylethylthio, insbesondere Methylthio;
C₁-C₄-Halogenalkylthio wie Difluromethylthio, Trifluormethylthio, Chlordifluormethylthio, 1-Fluorethylthio, 2-Fluorethylthio, 2,2-Difluorethylthio, 2,2,2-Trifluorethylthio, 2-Chlor-2,2-difluorethylthio, 2,2-Dichlor-2-fluorethylthio, 2,2,2-Trichlorethylthio und Pentafluorethylthio, insbesondere Trichlormethylthio;
Di-C₁-C₄-Alkylamino wie Dimethylamino, Diethylamino, Dipropylamino, Dibutylamino, N-Methyl-N-ethylamino, N-Methyl-N-propylamino, N-Methyl-N-1-methylethylamino, N-Methyl-N-1,1-dimethylethylamino, Di-1-Methylethylamino, N-Ethyl-N-1-methylethylamino und N-Ethyl-N-1,1-dimethylethylamino;
Phenyl, Phenylthio, Phenoxy, wobei die Phenylreste ihrerseits eine bis fünf Halogenatome wie Fluor, Chlor, Brom und Iod, insbesondere Fluor und Chlor,
und /oder eine bis drei der folgenden Gruppen tragen können:
Cyano oder Nitro;
C₁-C₄-Alkyl, wie vorstehend genannt, insbesondere Methyl, Ethyl, Propyl, 1-Methylpropyl und 1,1-Dimethylpropyl;
C₁-C₄-Halogenalkyl, insbesondere Trifluormethyl;
C₁-C₄-Alkoxy, wie vorstehend genannt, insbesondere Methoxy und Ethoxy;
C₁-C₄-Halogenalkoxy, wie vorstehend genannt, insbesondere Trifluormethoxy;
- R: Wasserstoff,
C₁-C₁₂-Alkyl, C₃-C₁₂-Alkenyl, C₃-C₁₂-Alkinyl, C₃-C₈-Cycloalkyl, jeweils wie vorstehend für R¹ im allgemeinen und im besonderen genannt
C₃-C₈-Alkenyloxy wie 2-Propenyloxy, 2-Butenyloxy, 3-Butenyloxy, 1-Methyl-2-propenyloxy, 2-Methyl-2-propenyloxy, 2-Pentenyloxy, 3-Pentenyloxy, 4-Pentenyloxy, 3-Methyl-2-butenyloxy, 1-Methyl-2-butenyloxy, 2-Methyl-2-butenyloxy, 1-Methyl-3-butenyloxy, 2-Methyl-3-butenyloxy, 3-Methyl-3-butenyloxy, 1,1-Dimethyl-2-propenyloxy, 1,2-Dimethyl-2-propenyloxy, 1-Ethyl-2-propenyloxy, 2-Hexenyloxy, 3-Hexenyloxy, 4-Hexenyloxy, 5-Hexenyloxy, 1-Methyl-2-pentenyloxy, 2-Methyl-2-pentenyloxy, 3-Methyl-2-pentenyloxy, 4-Methyl-2-pentenyloxy, 1-Methyl-3-pentenyloxy, 2-Methyl-3-pentenyloxy, 3-Methyl-3-pentenyloxy, 4-Methyl-3-pentenyloxy, 1-Methyl-4-pentenyloxy, 2-Methyl-4-pentenyloxy, 3-Methyl-4-pentenyloxy, 4-Methyl-4-pentenyloxy, 1,1-Dimethyl-2-butenyloxy, 1,1-Dimethyl-3-butenyloxy, 1,2-Dimethyl-2-butenyloxy, 1,2-Dimethyl-3-butenyloxy, 1,3-Dimethyl-2-butenyloxy, 1,3-Dimethyl-3-butenyloxy, 2,2-Dimethyl-3-butenyloxy, 2,3-Dimethyl-2-butenyloxy, 2,3-Dimethyl-4-butenyloxy, 1-Ethyl-2-butenyloxy, 1-Ethyl-3-butenyloxy,2-Ethyl-2-butenyloxy, 2-Ethyl-3-butenyloxy, 1,1,2-Trimethyl-2-propenyloxy, 1-Ethyl-1-methyl-2-propenyloxy und Ethyl-2-methyl-2-propenyloxy, insbesondere 2-Propenyloxy;
Di-C₁-C₄-Alkylamino, wie vorstehend bei R¹ genannt,
wobei die genannten organischen Gruppen ein bis fünf Halogenatome wie Fluor, Chlor, Brom und Iod, vorzugsweise Fluor, Chlor und Brom, insbesondere Fluor und Chlor, und/oder einen bis drei der folgenden Reste tragen können:
Cyano,
C₃-C₈-Cycloalkyl, C₁-C₄-Alkoxy, C₁-C₄-Halogenalkoxy, C₁-C₄-Alkylthio, C₁-C₄-Halogenalkylthio und Di-C₁-C₄-Alkylamino, jeweils wie vorstehend im einzelnen angegeben,
Phenyl, Phenylthio, Phenoxy, wobei die Phenylreste ihrerseits eine bis fünf Halogenatome wie Fluor, Chlor, Brom und Iod, insbesondere Fluor und Chlor,
und/oder eine bis drei der folgenden Gruppen tragen können:
Cyano oder Nitro,
C₁-C₄-Alkyl, C₁-C₄-Alkoxy, jeweils wie vorstehend genannt;
R¹ und R gemeinsam eine C₂-C₆-Alkylenkette, welche durch Sauerstoff, Schwefel oder N-Methyl unterbrochen sein kann wie -(CH₂)₂, -(CH₂)₃-, -(CH₂)₄-, -(CH₂)₅-, -(CH₂)₆-, -CH₂-O-CH₂-, -CH₂-CH₂-O-CH₂-CH₂-, -CH₂-S-CH₂-, -CH₂-CH₂-S-CH₂-CH₂-, -CH₂-N(CH₃)-CH₂-CH₂-, insbesondere -(CH₂)₅- und -CH₂-CH₂-O-CH₂-CH₂-;
- X: Sauerstoff oder Schwefel;
- Q: ein fünf- oder sechsgliedriger heteroaromatischer Ring enthaltend 1 - 3 Stickstoffatome und/oder ein Sauerstoff- oder Schwefelatom als Heteroatome wie Pyridin, Pyrimidin, Pyrazin, Pyridazin, Triazin, Thiophen, Furan, Pyrrol, Oxazol, Isoxazol, Thiazol, Isothioazol, Pyrazol, Imidazol Triazol, Oxadiazol und Thiadiazol, der über benachbarte Kohlenstoffatome mit dem Hydroxypyridonring verknüpft ist und ein bis drei der folgenden Reste tragen kann:
Halogen wie Fluor, Chlor, Brom und Iod, insbesondere Fluor und Chlor,
Cyano und/oder Nitro,
C₁-C₄-Alkyl, wie vorstehend genannt, insbesondere Methyl, Ethyl, Propyl, 1-Methylethyl und 1,1-Dimethylethyl,
C₁-C₄-Alkoxy, wie vorstehend genannt, insbesondere Methoxy und Ethoxy,
C₁-C₄-Alkylthio, wie vorstehend genannt, insbesondere Methylthio und Ethylthio,
C₁-C₄-Alkoxyal)cyl, insbesondere Methoxymethyl, Ethoxymethyl und Methoxyethyl,
C₃-C₈-Cycloalkyl wie vorstehend genannt, insbesondere Cyclopropyl und Cyclohexyl,
C₂-C₄-Alkenyl, insbesondere Ethenyl und Propenyl,
Phenyl,
Phenyl-C₁-C₄-alkyl, insbesondere Benzyl und Phenylethyl, wobei die Phenylgruppen ihrerseits ein bis drei der folgenden Gruppen tragen können: Halogen,C₁-C₄-Alkyl, C₁-C₄-Alkoxy, C₁-C₄-Alkylthio, jeweils wie vorstehend genannt,
C₁-C₄-Halogenalkyl, insbesondere Trifluormethyl
C₁-C₄-Halogenalkoxy, insbesondere Trifluormethoxy.

Als Salze der Verbindungen I kommen landwirtschaftlich brauchbare Salze, beispielsweise Alkalimetallsalze, insbesondere das Kalium- oder Natriumsalz, Erdalkalimetallsalze, insbesondere das Calcium-, Magnesium-oder Bariumsalz, Mangan-, Kupfer-, Zink- oder Eisensalze sowie Ammonium-, Phosphonium-, Tetraalkylammonium- salze, Benzyltrialkylammoniumsalze, Trialkylsulfoniumsalze oder Trialkylsulfoxoniumsalze in Betracht.

Beispiele für besonders bevorzugte Verbindungen der allgemeinen Formel I sind in den folgenden Tabellen zusammengestellt:

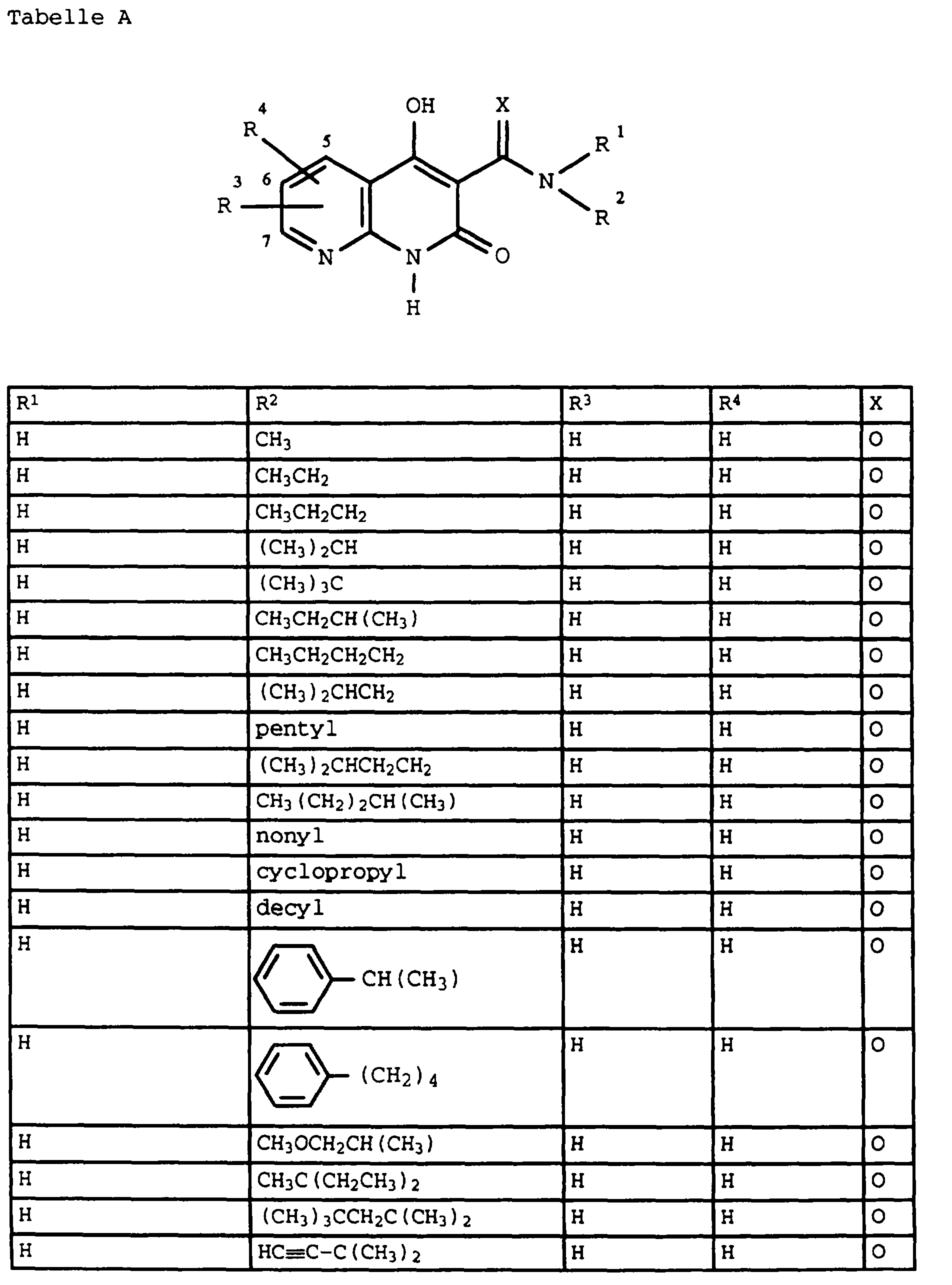

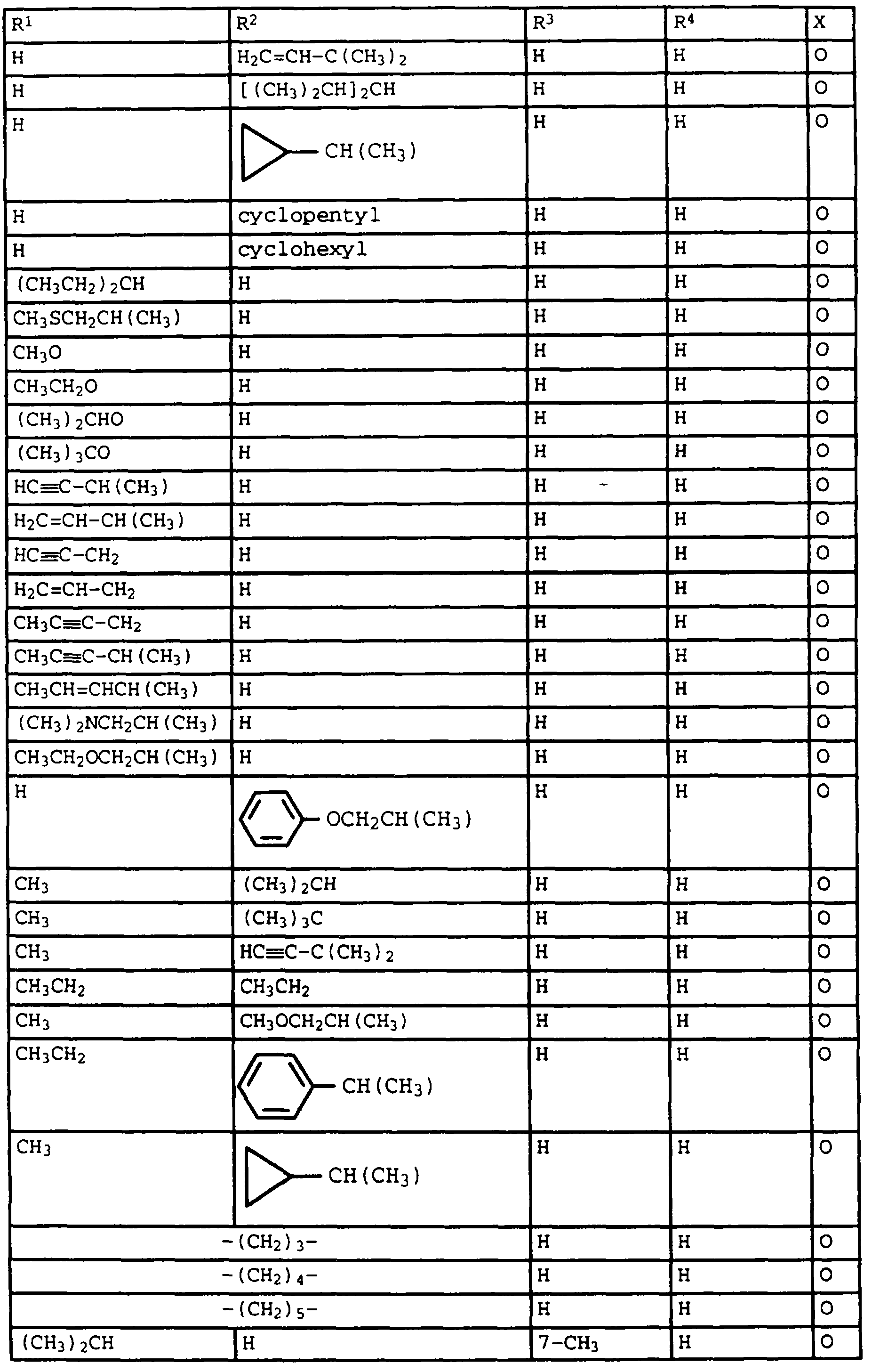

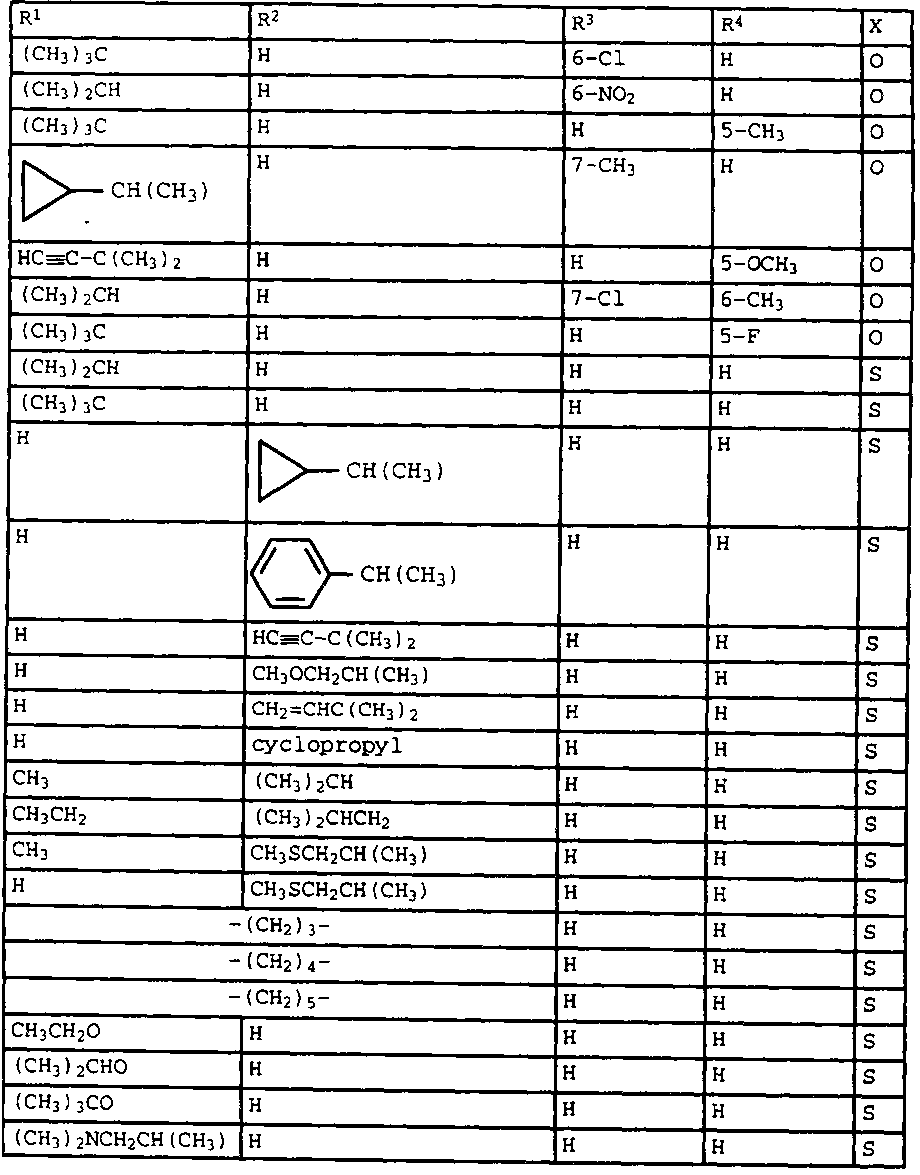

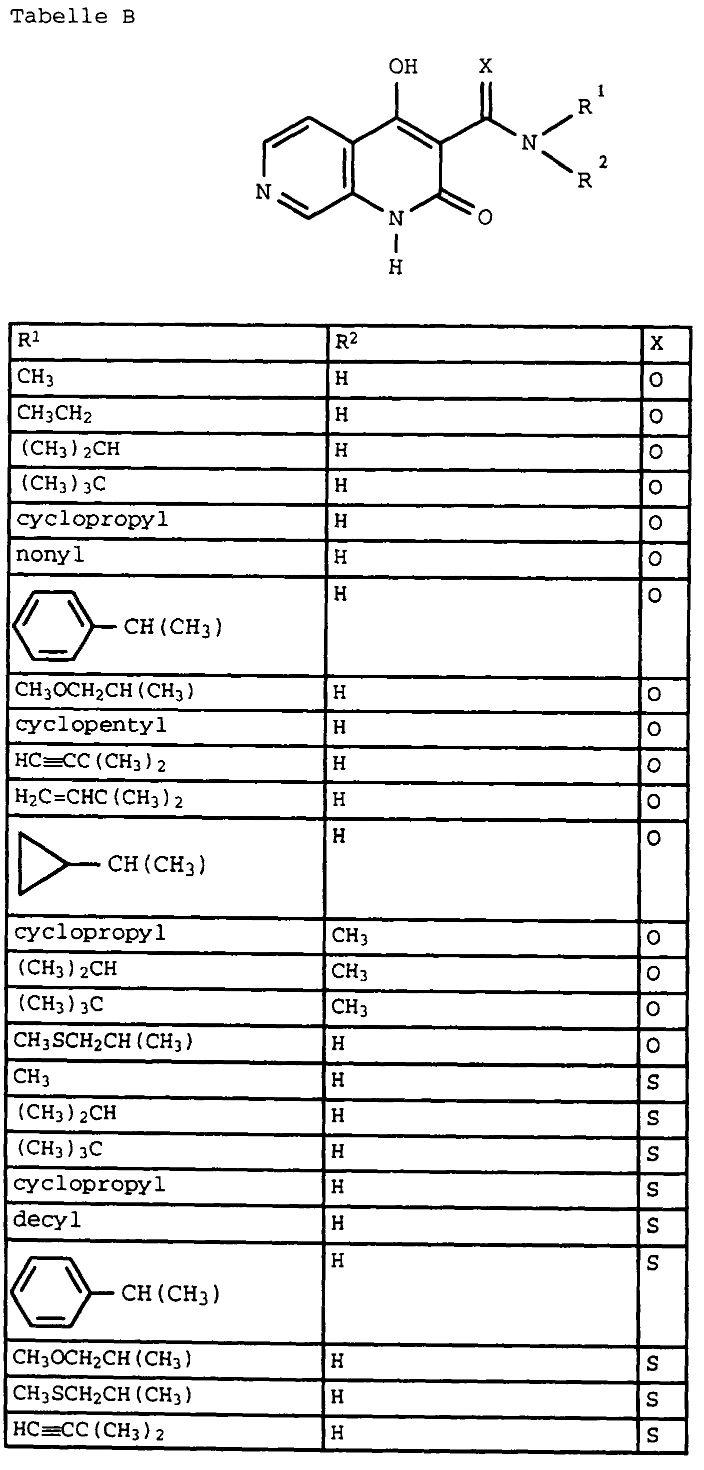

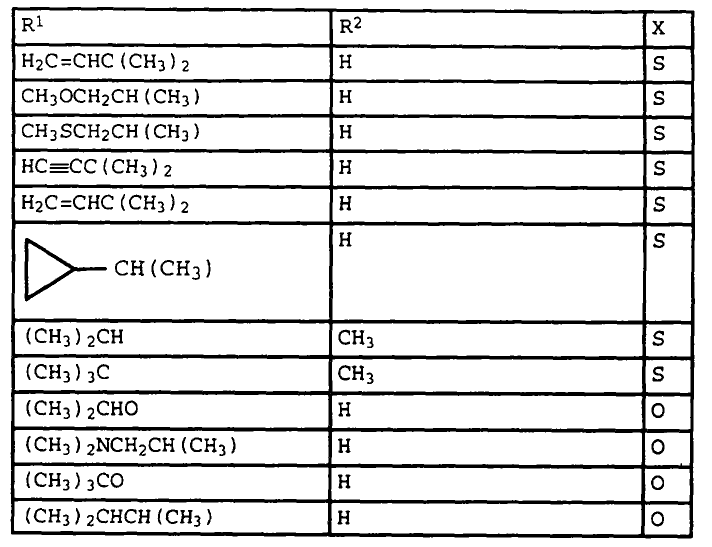

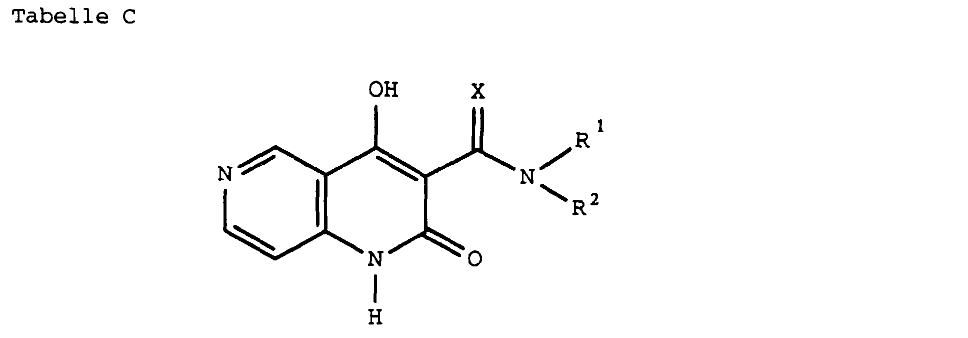

worin R¹, R und X die in Tabelle A im einzelnen genannte Bedeutung haben. 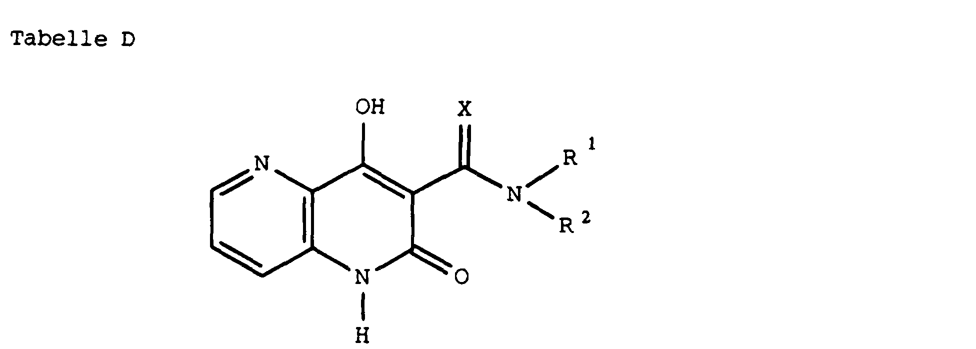 worin R¹, R und X die in Tabelle A im einzelnen genannte Bedeutung haben.

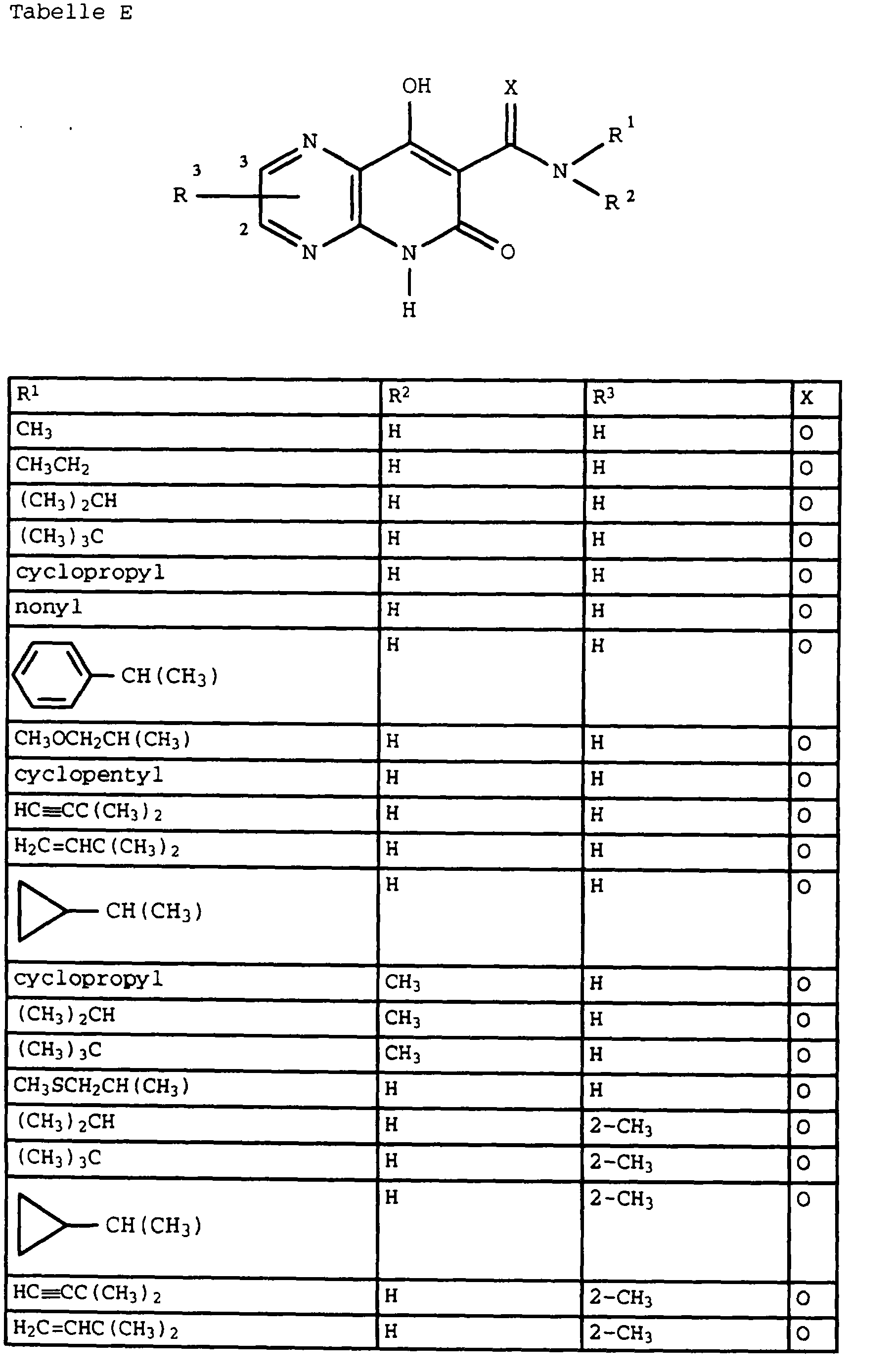

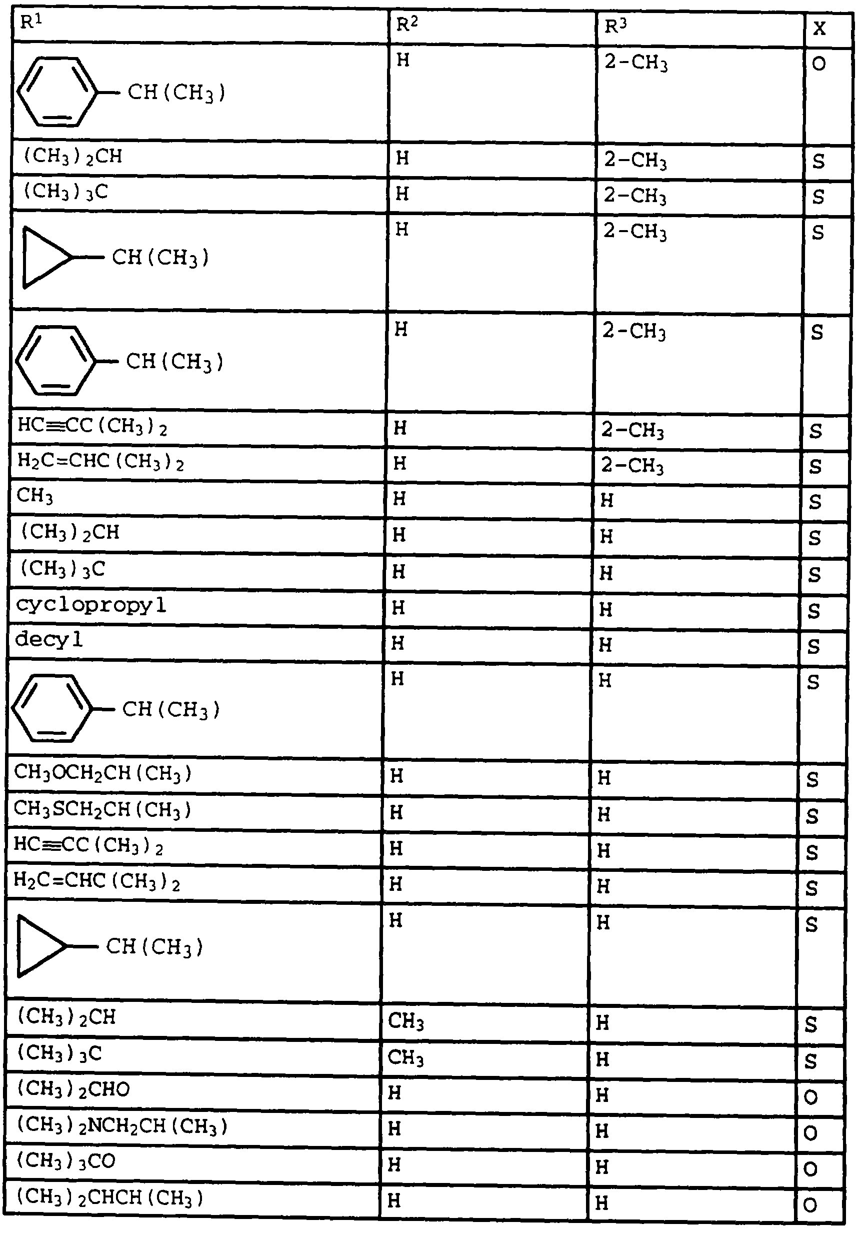

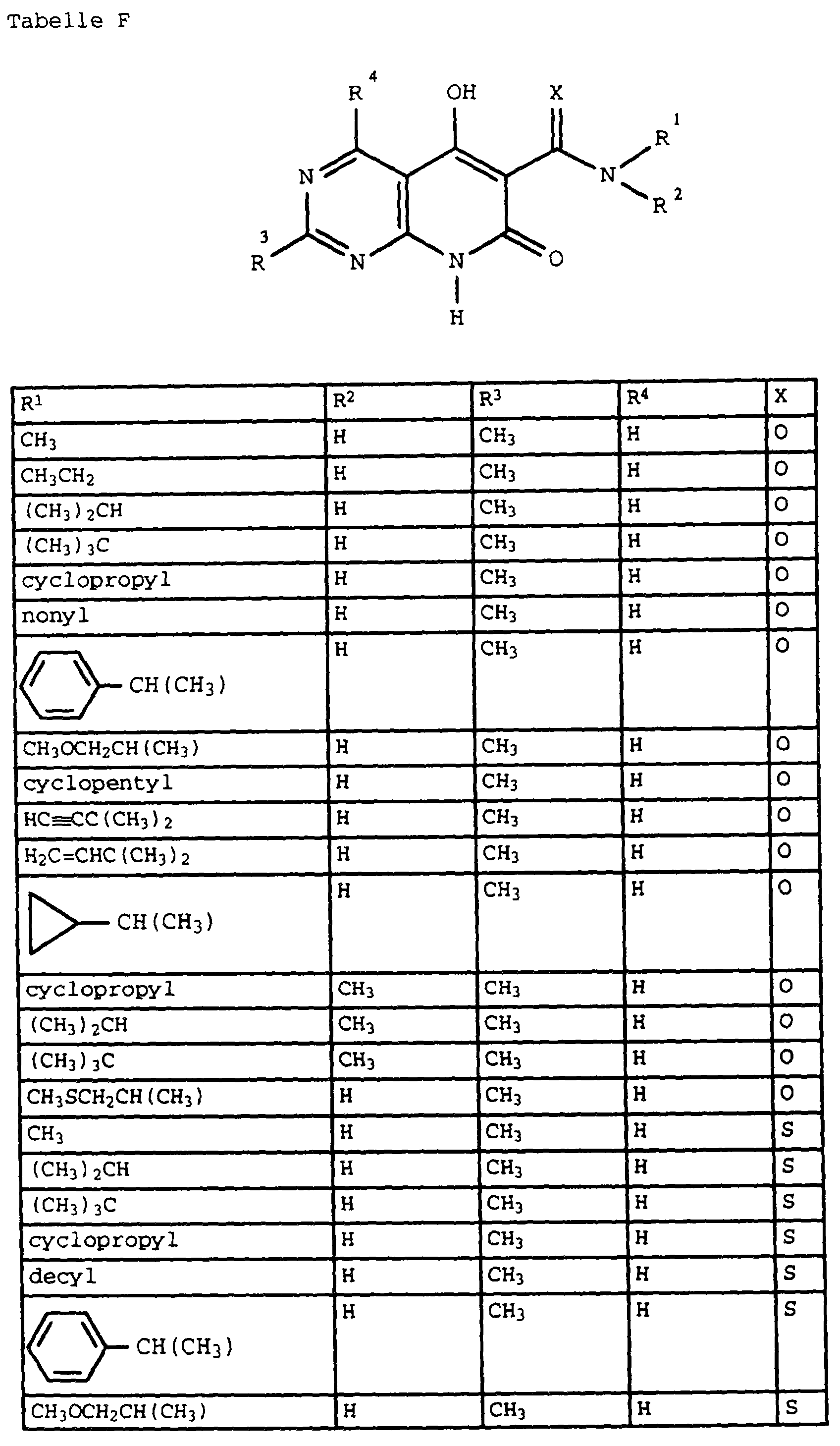

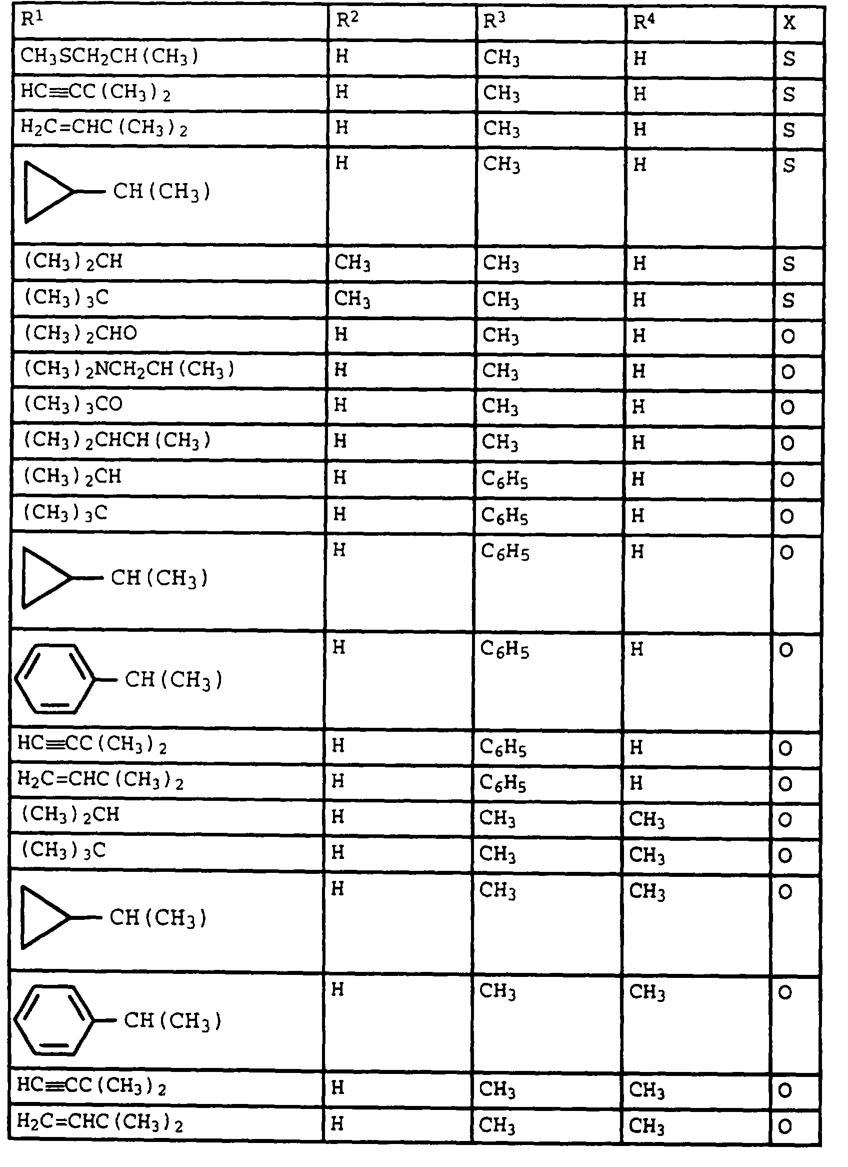

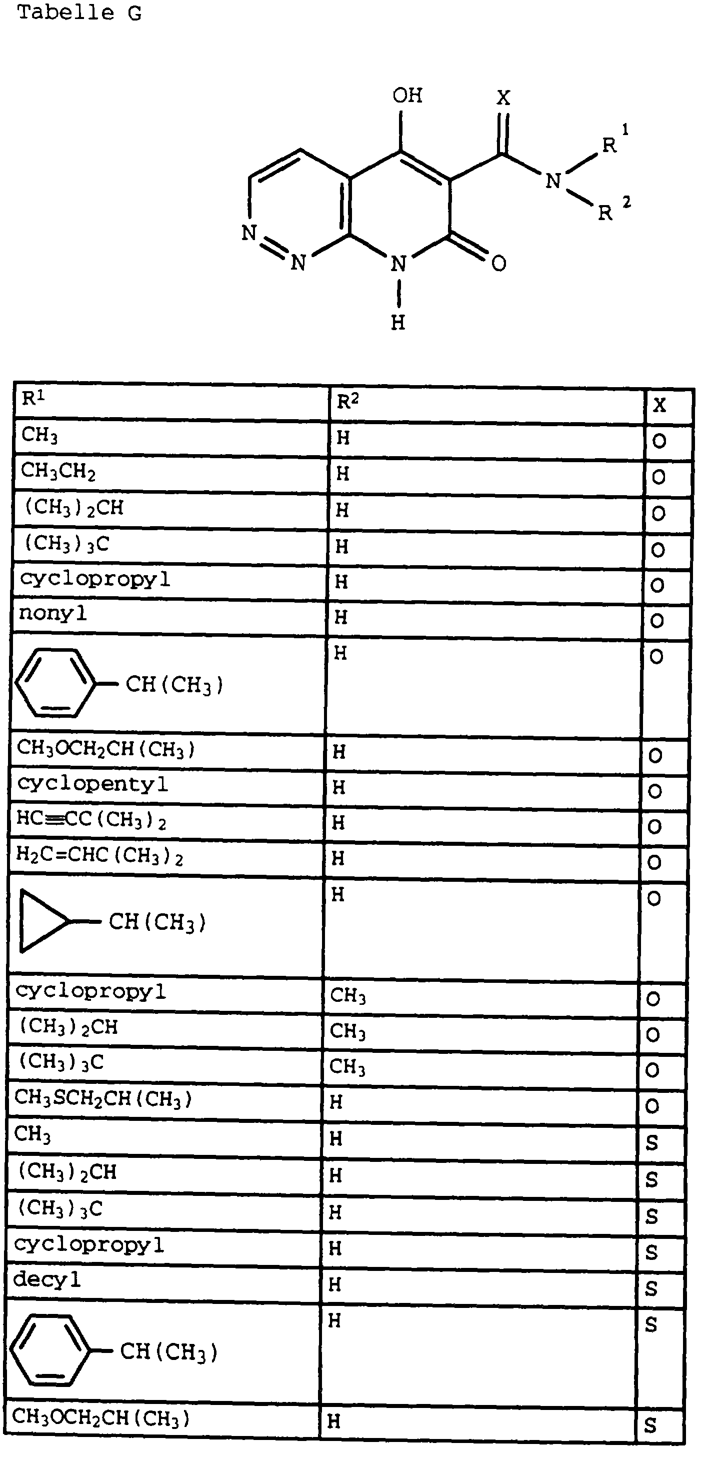

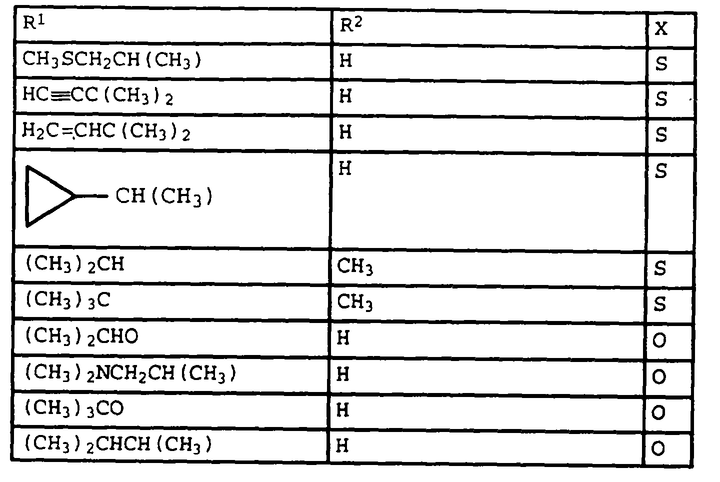

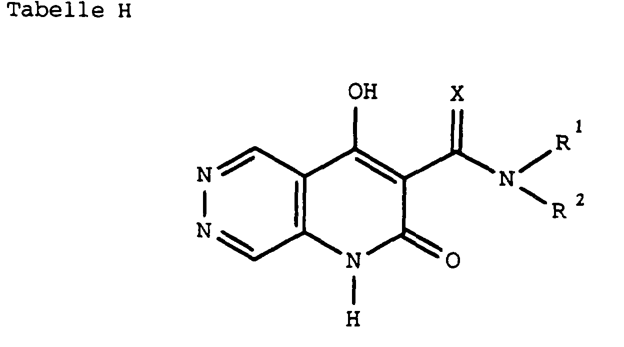

worin R¹, R und X die in Tabelle G im einzelnen genannte Bedeutung haben. 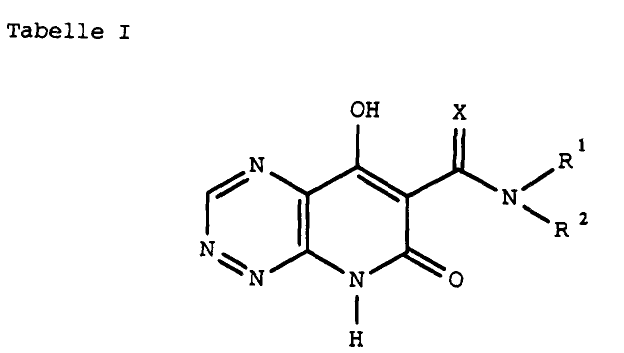 worin R¹, R und X die in Tabelle G im einzelnen genannte Bedeutung haben.

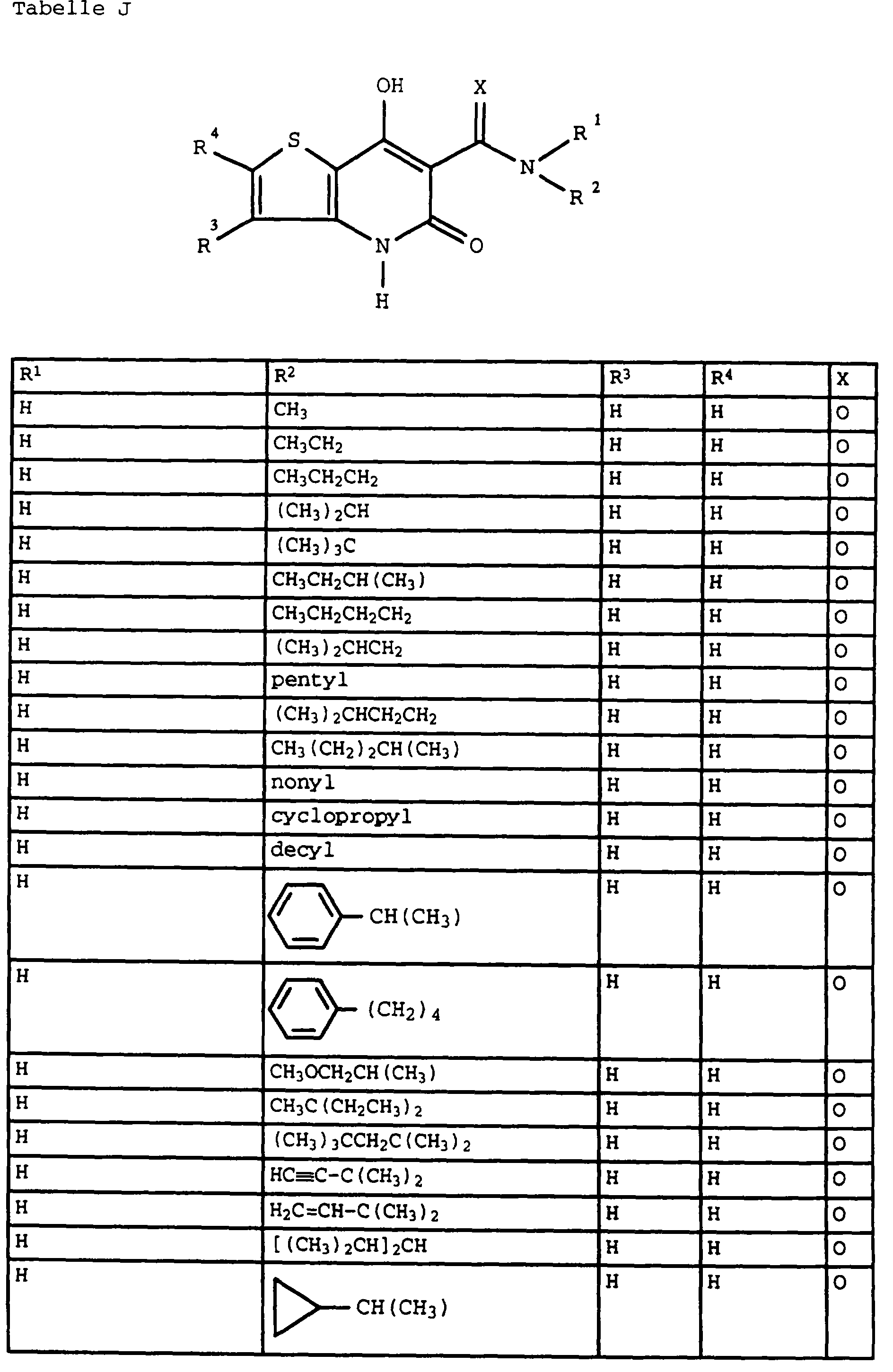

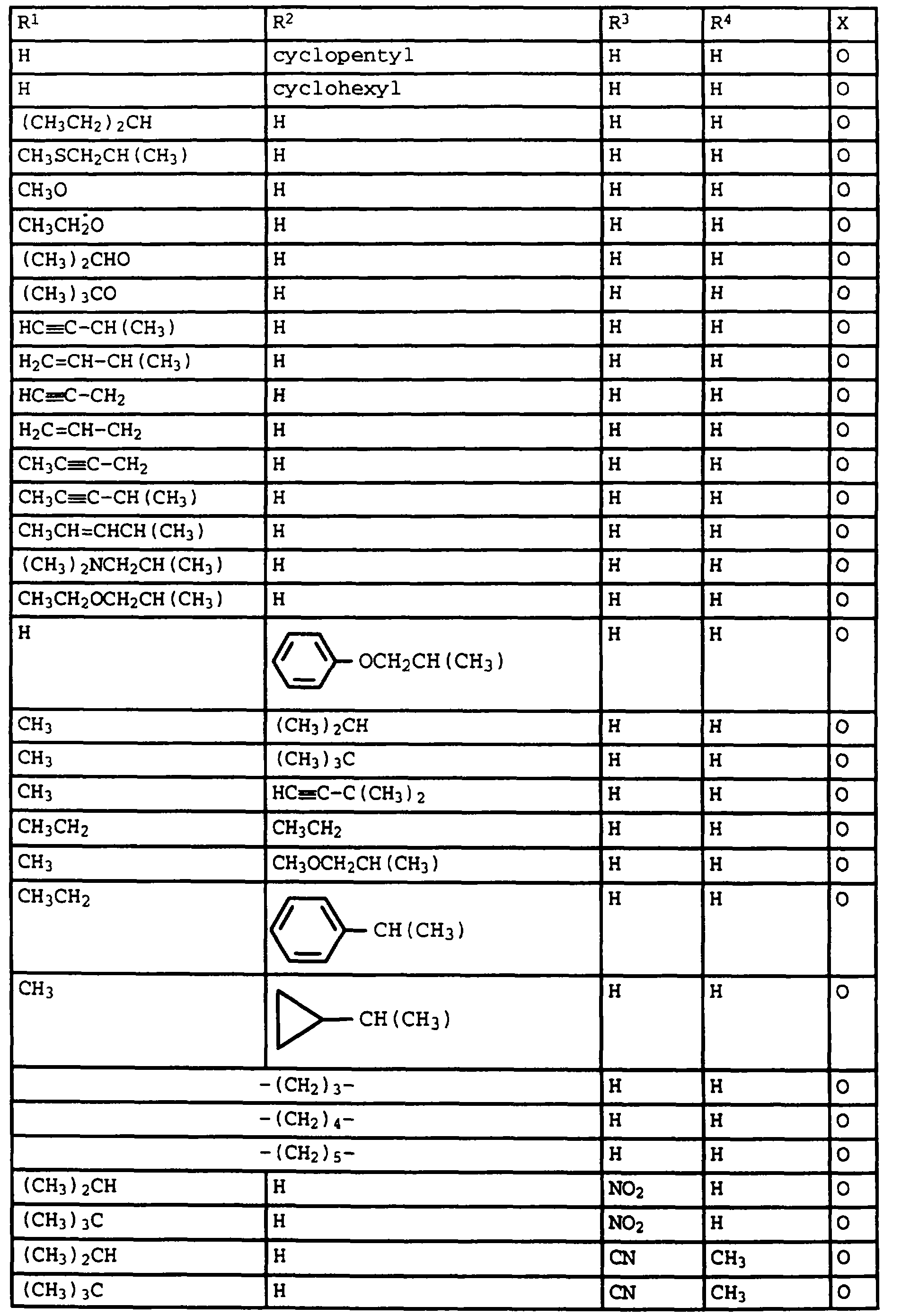

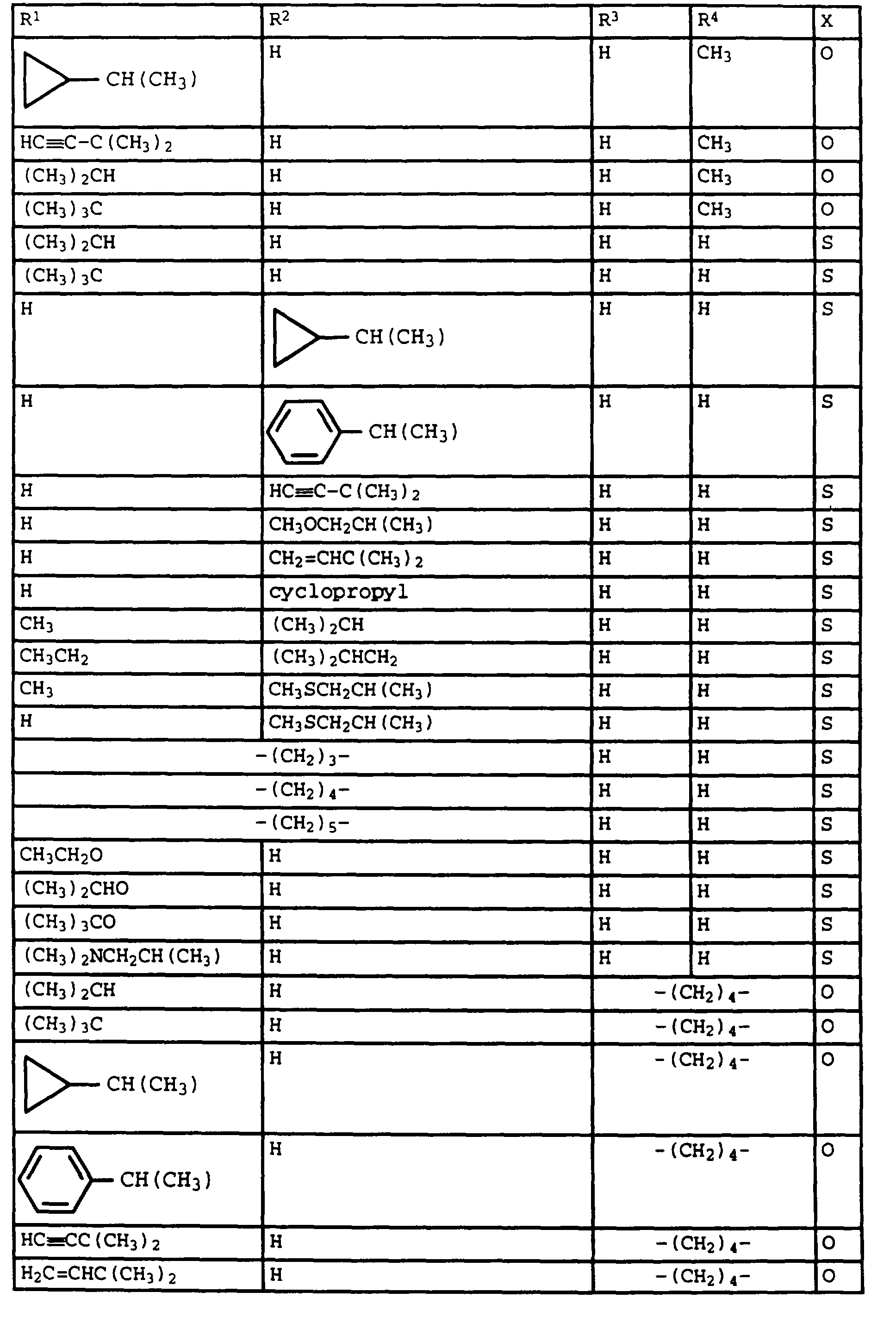

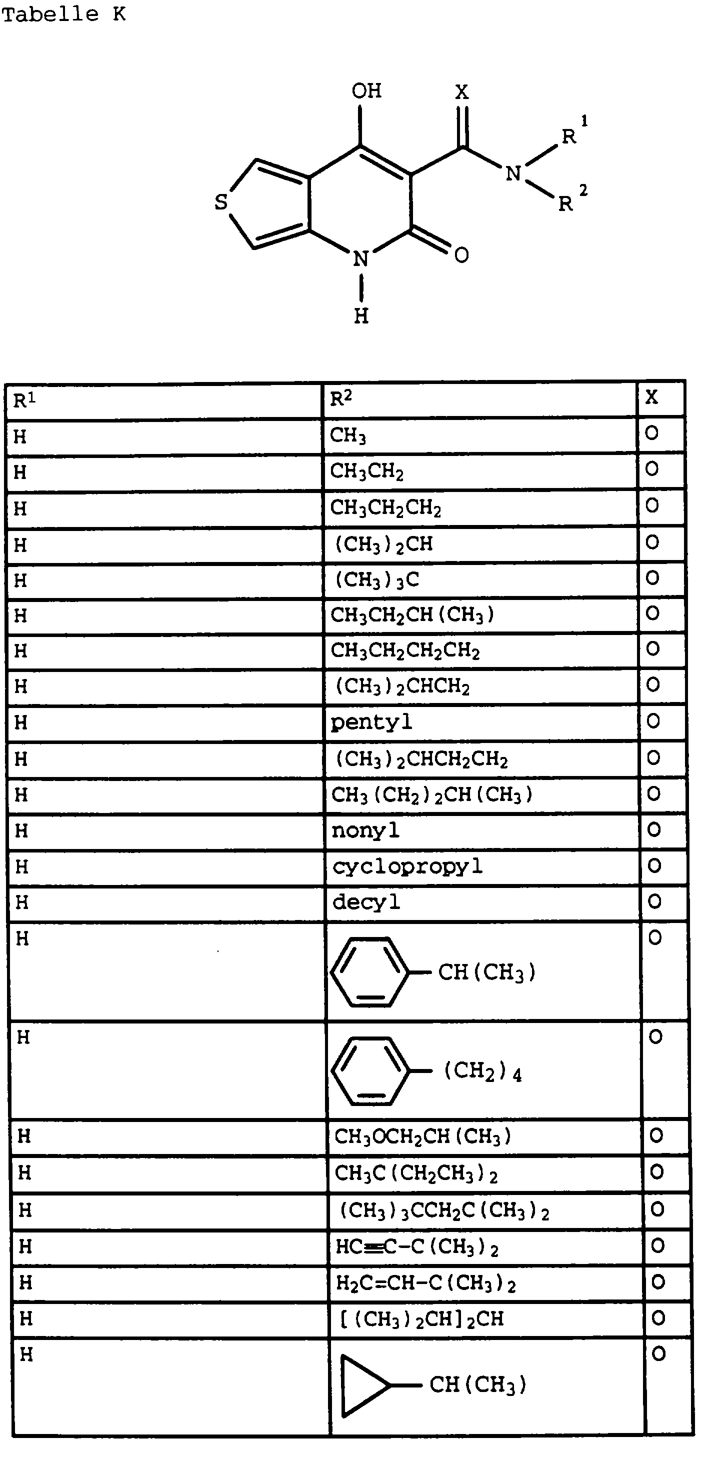

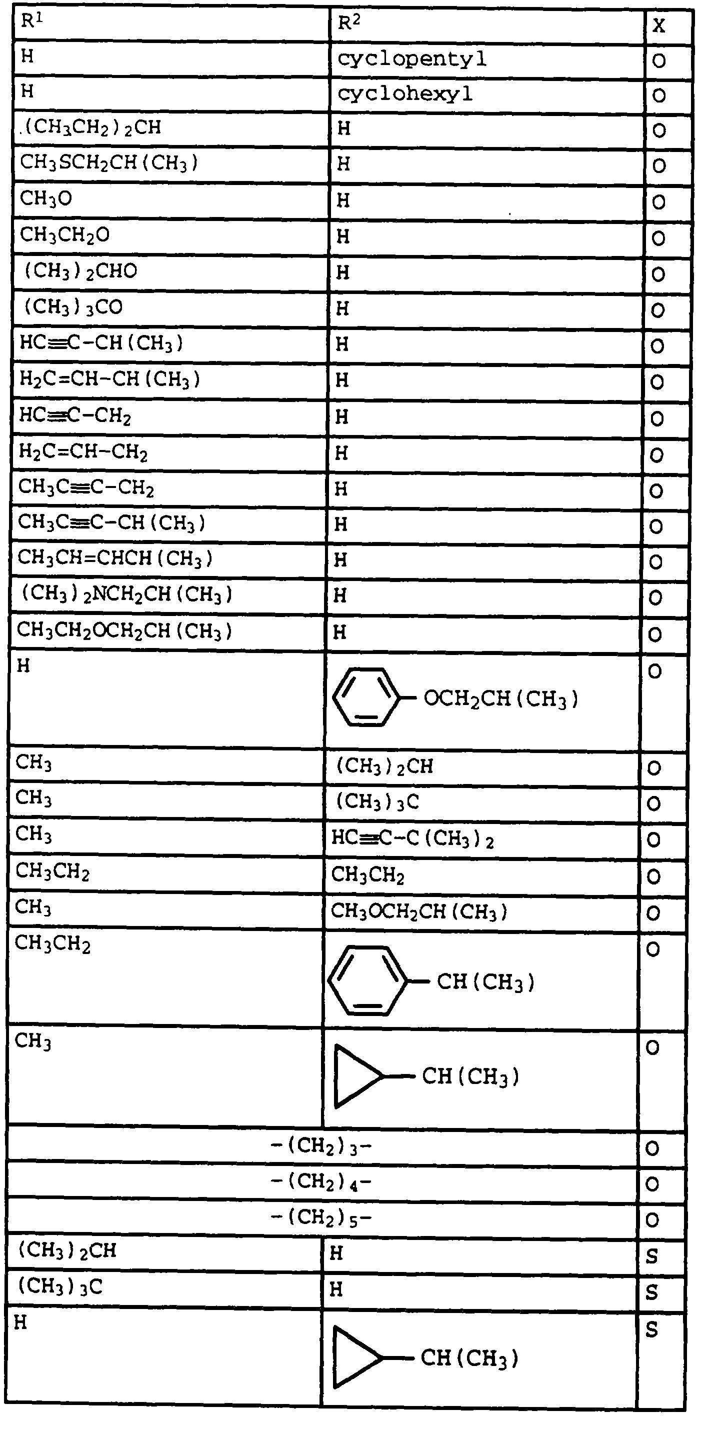

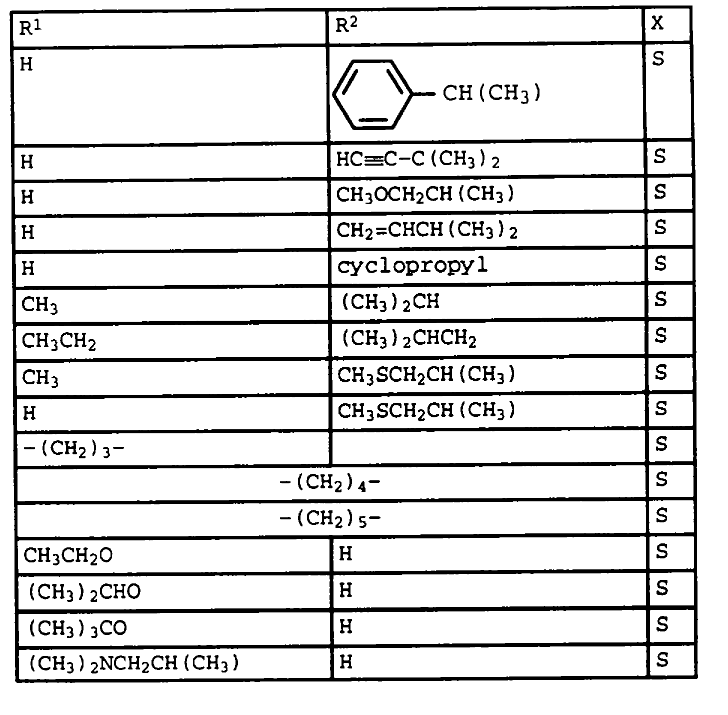

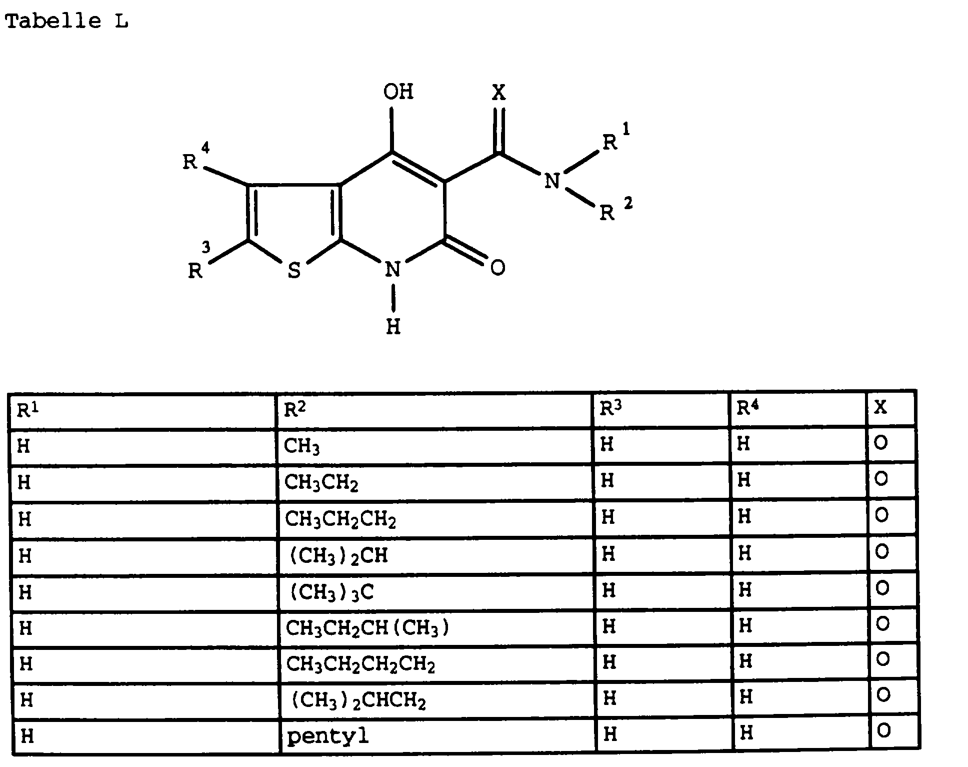

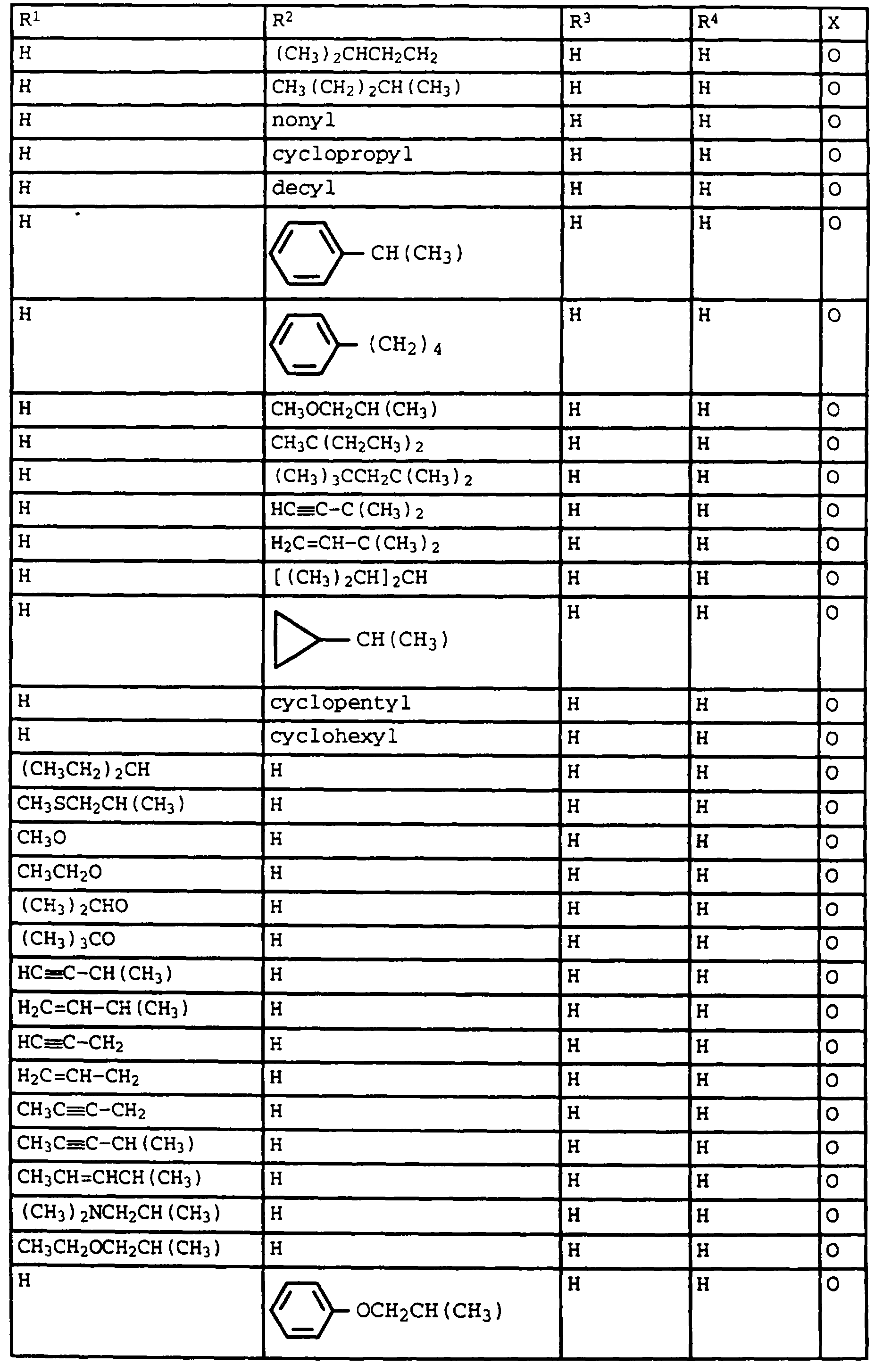

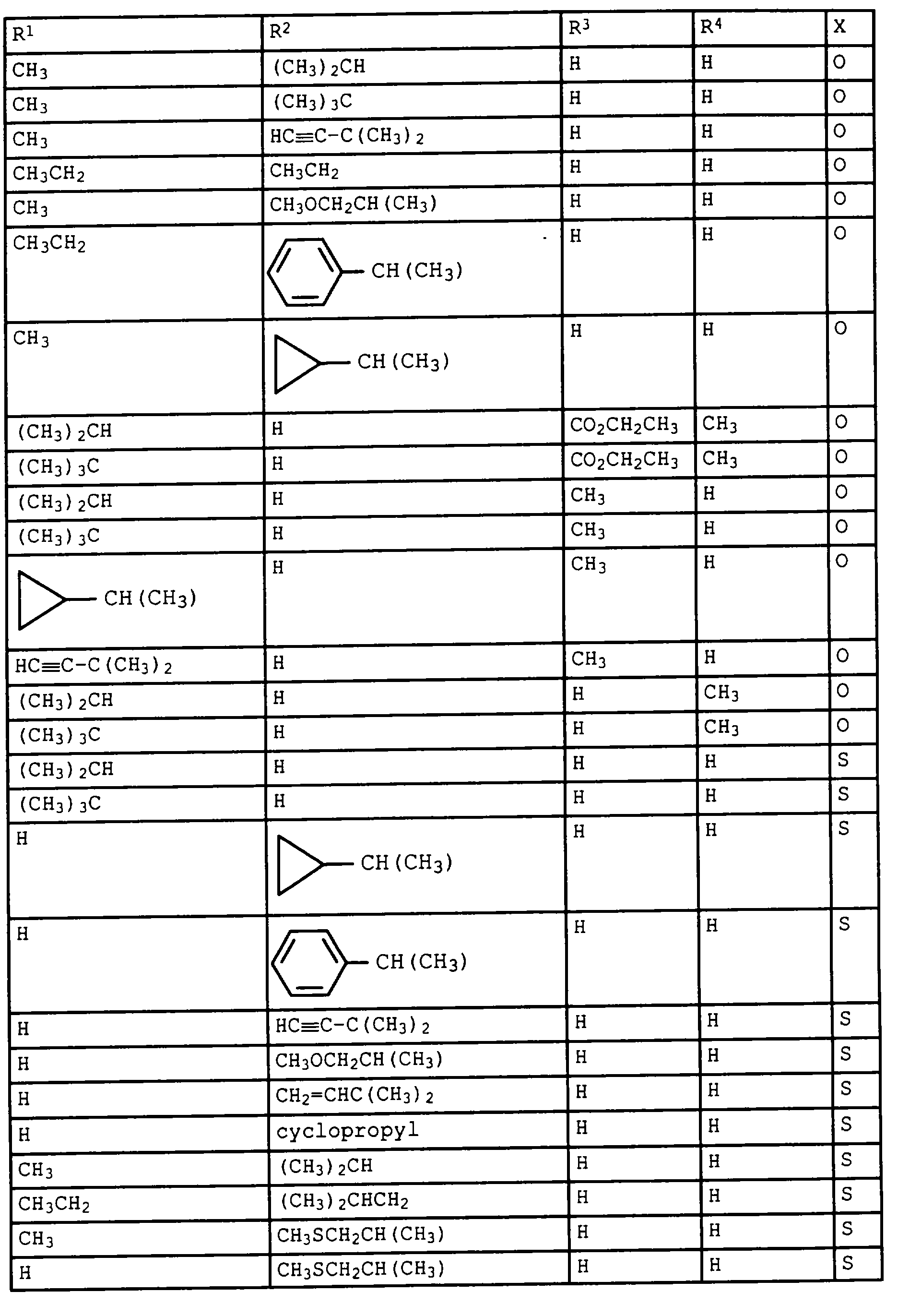

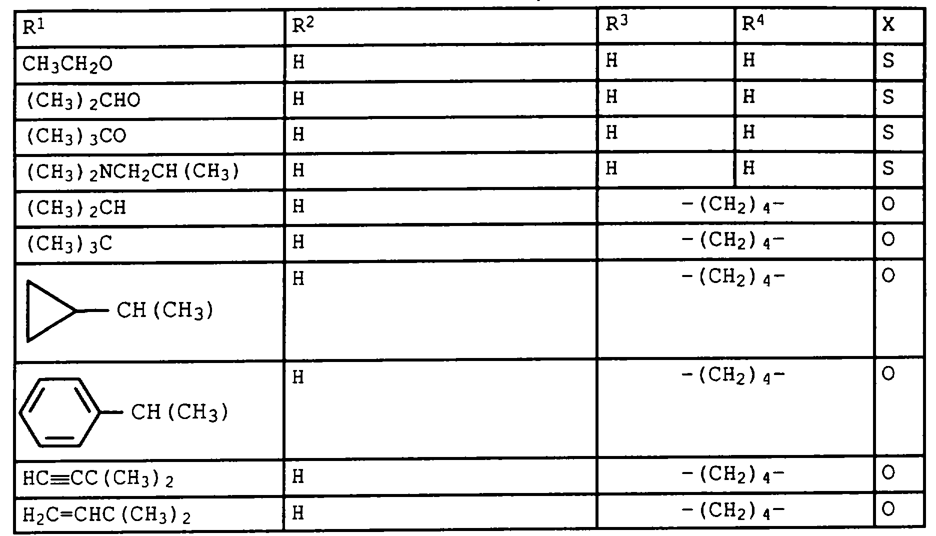

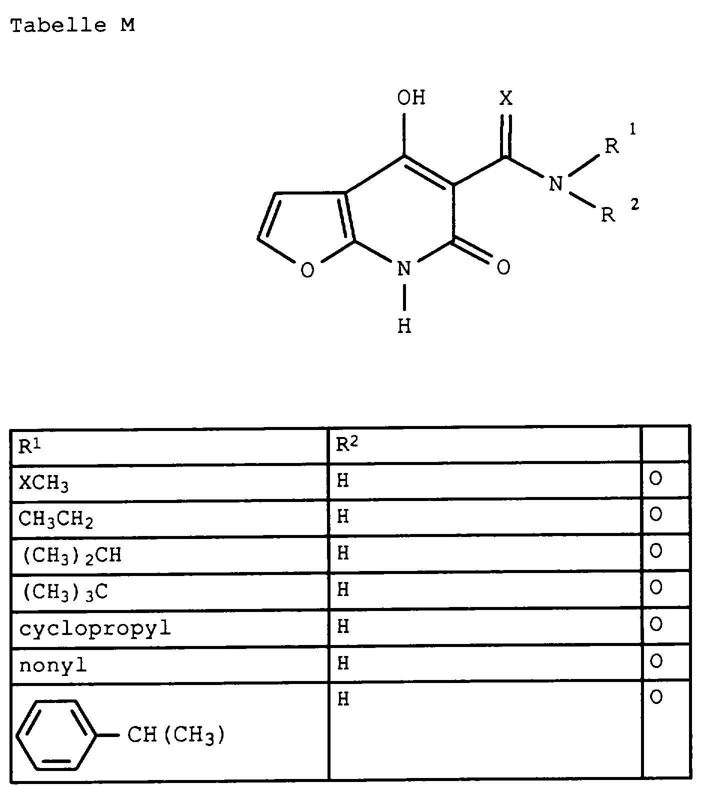

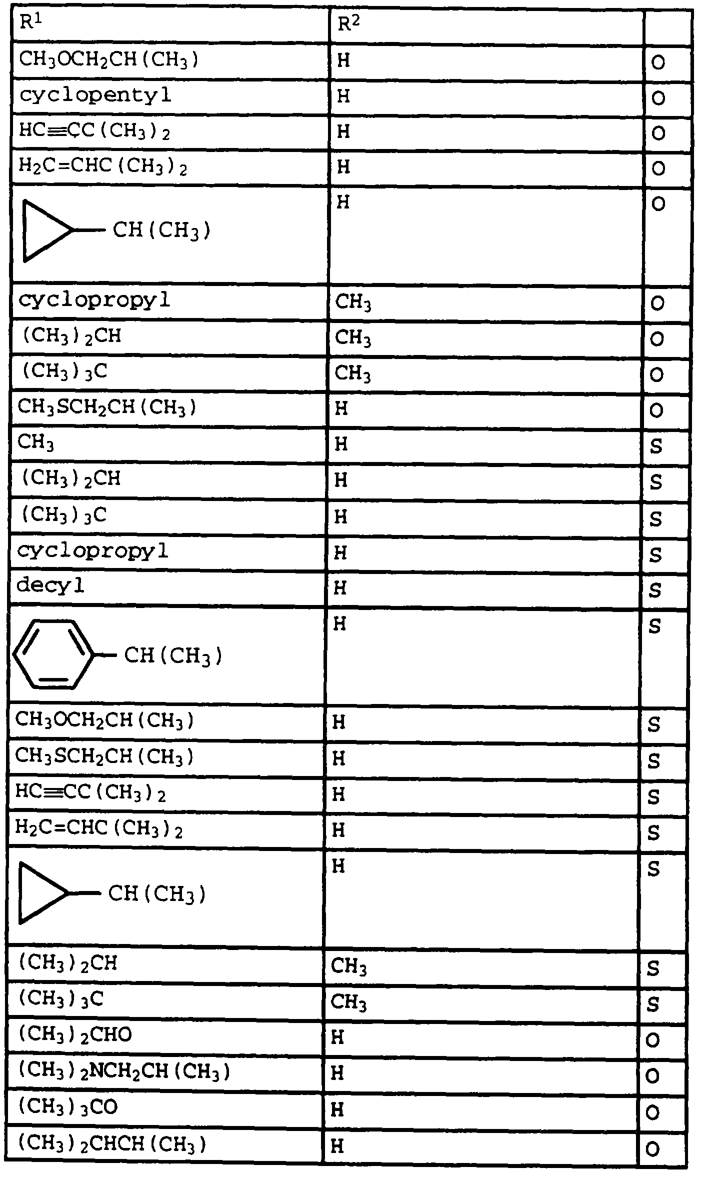

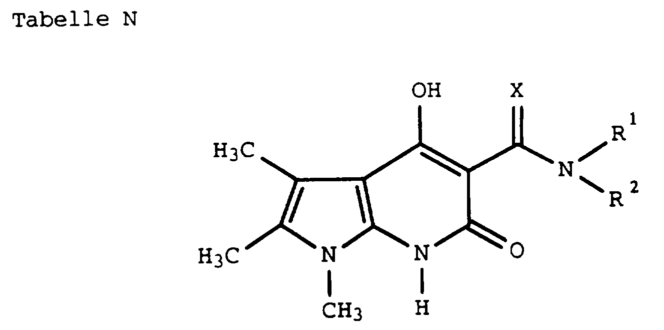

worin R¹, R und X die in Tabelle M im einzelnen genannte Bedeutung haben.

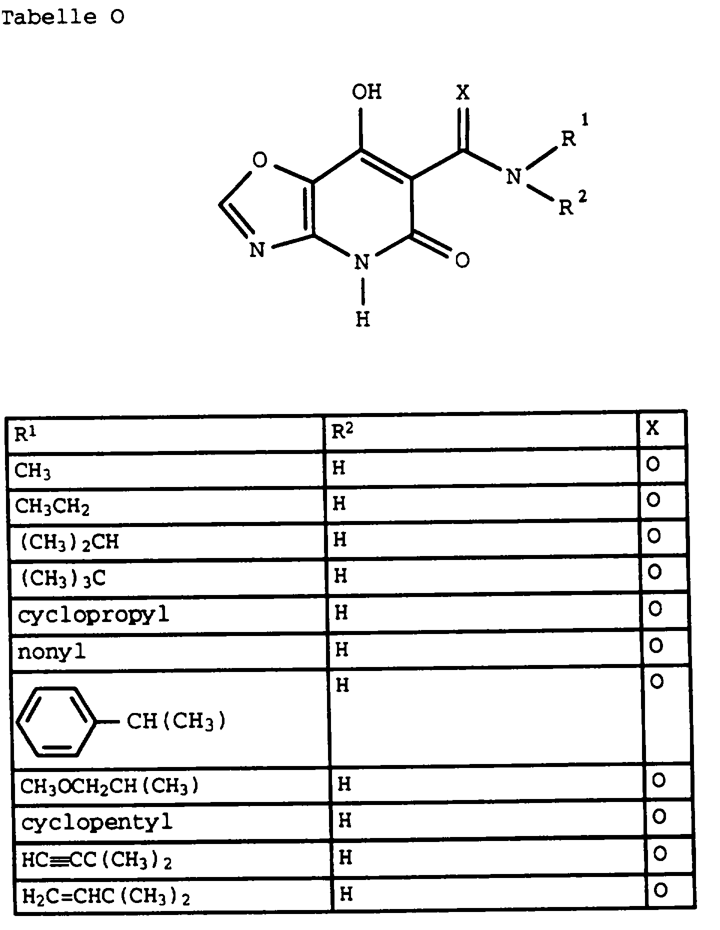

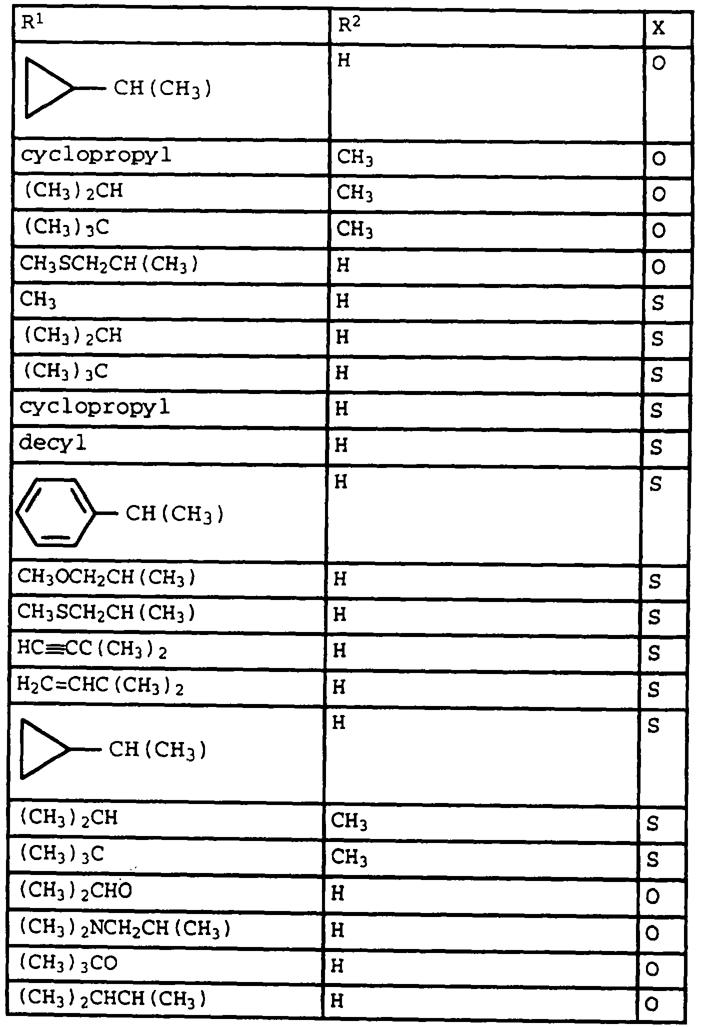

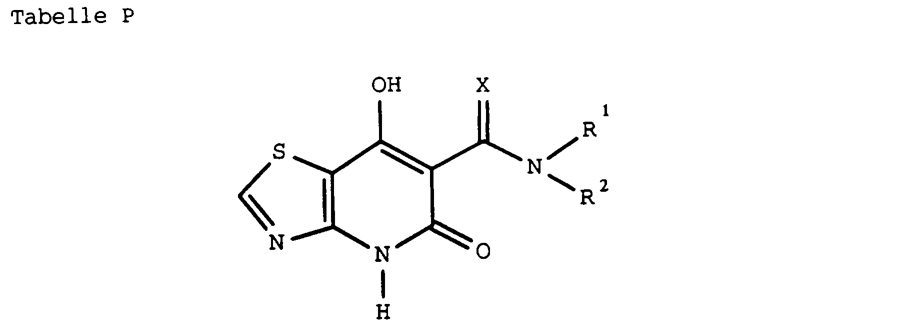

worin R¹, R und X die in Tabelle O im einzelnen genannte Bedeutung haben.

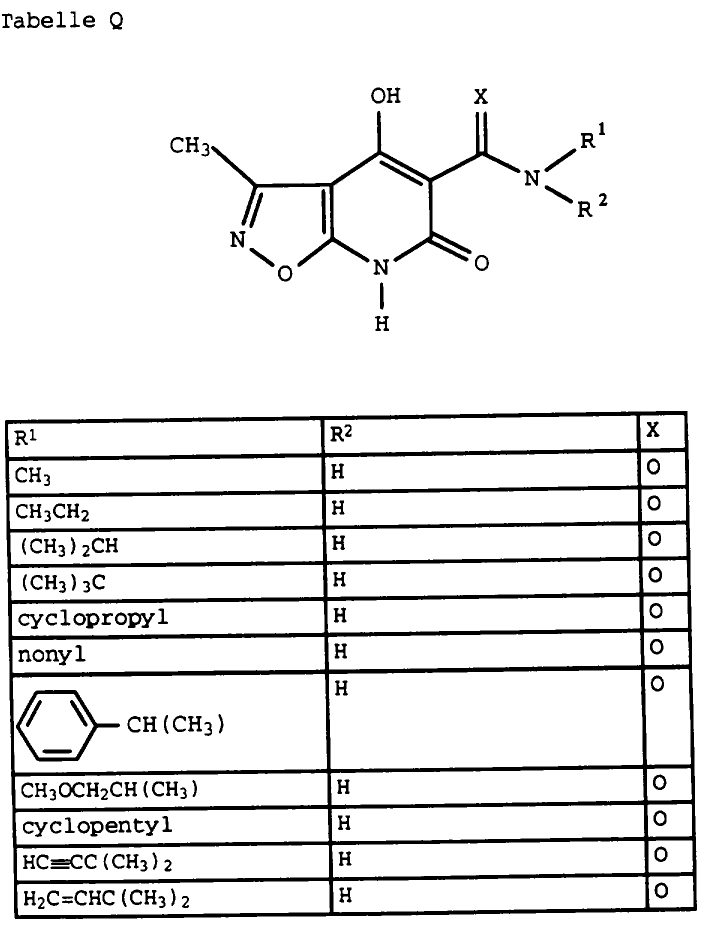

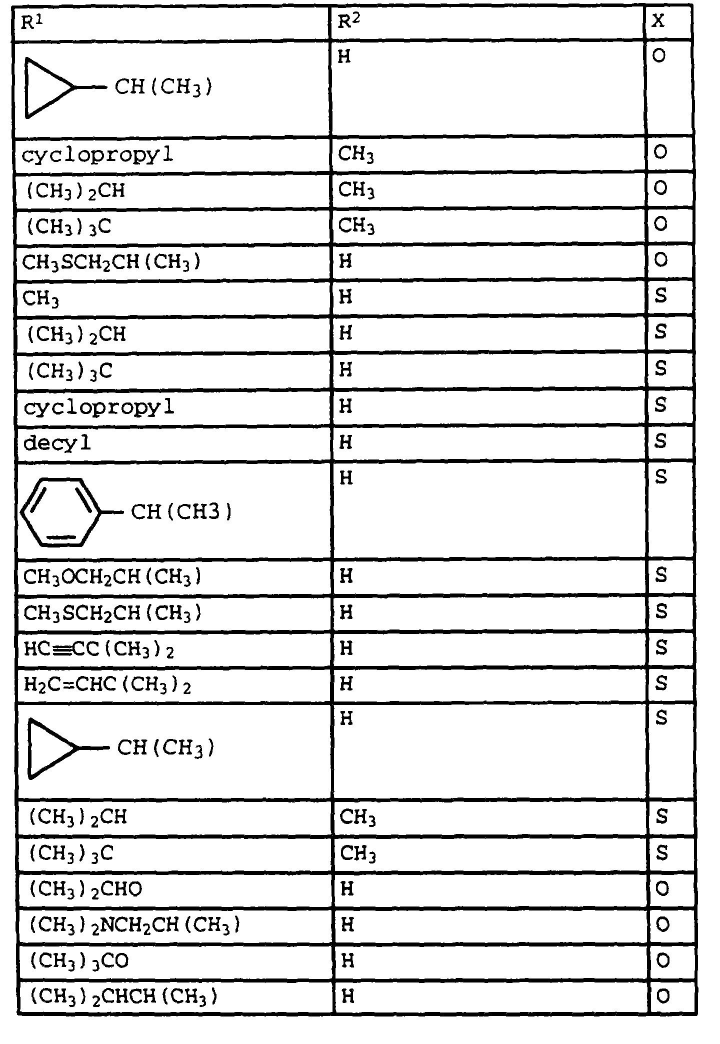

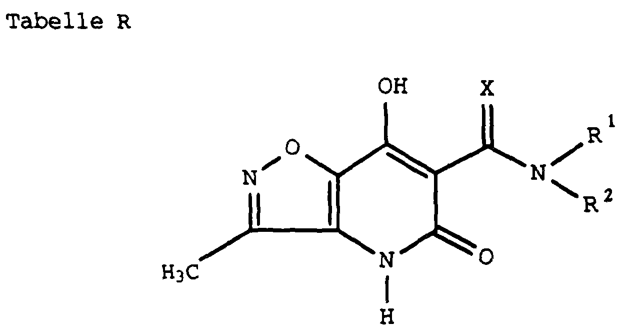

worin R¹, R und X die in Tabelle Q im einzelnen genannte Bedeutung haben. 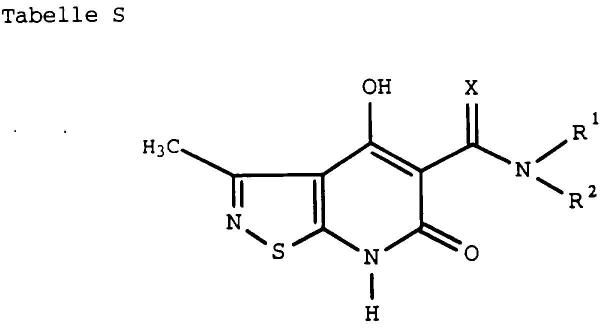 worin R¹, R und X die in Tabelle Q im einzelnen genannte Bedeutung haben.

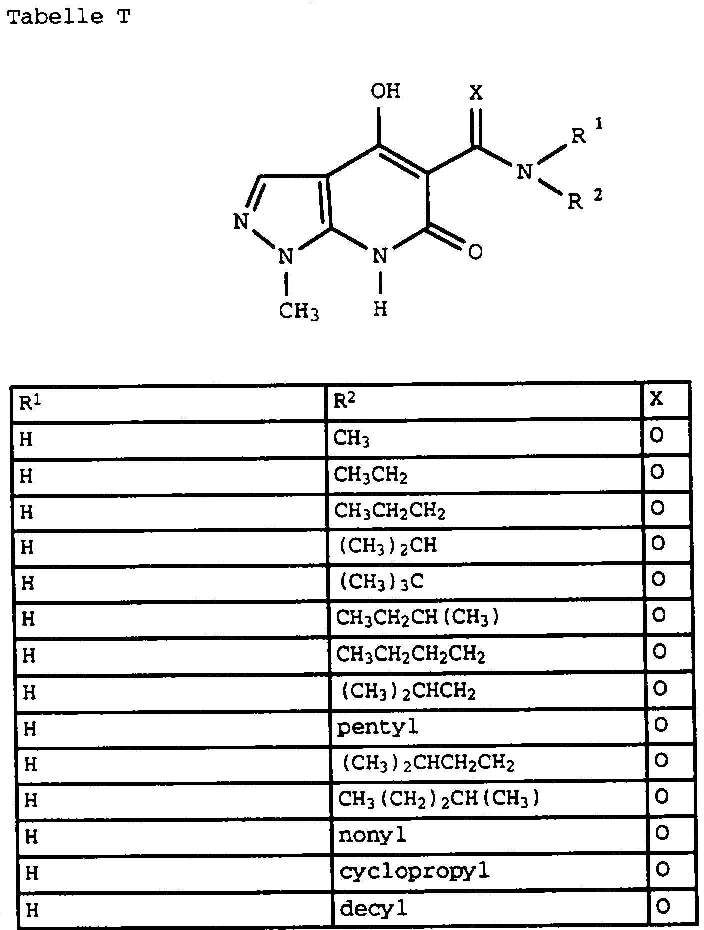

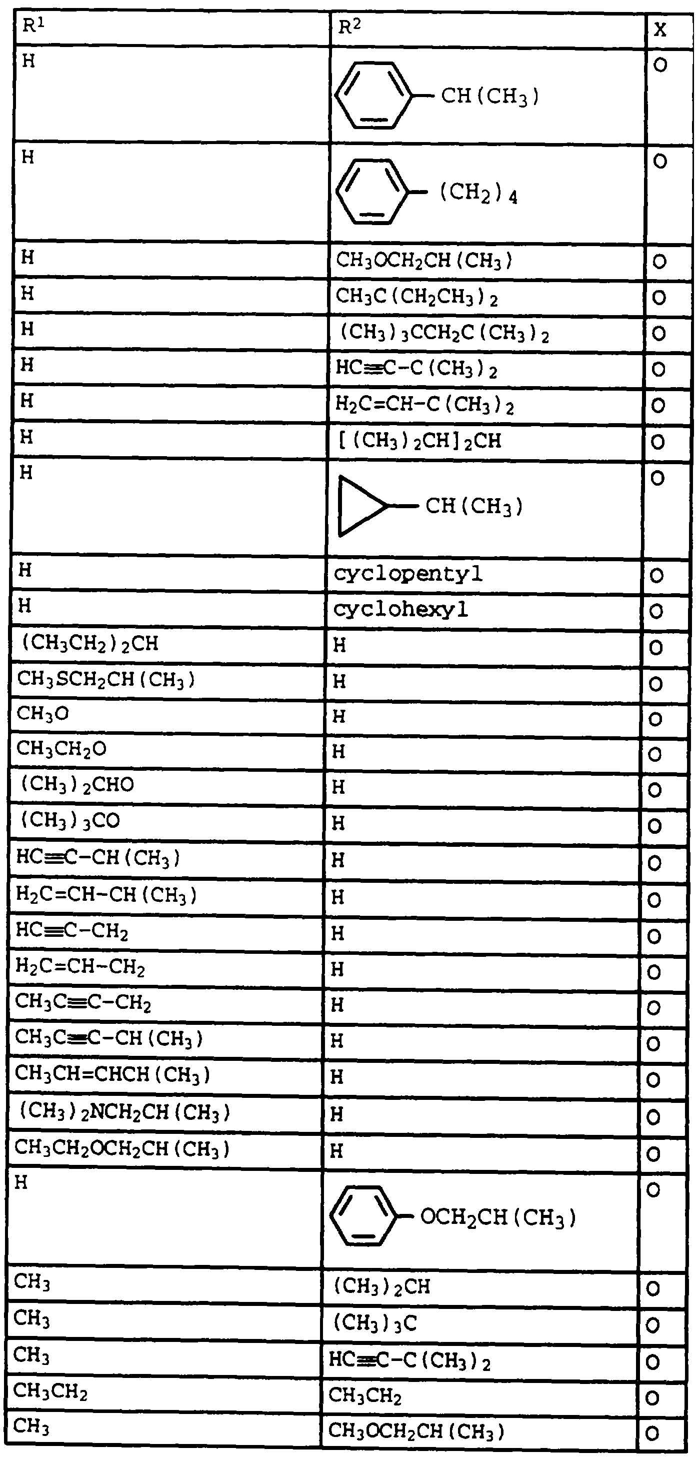

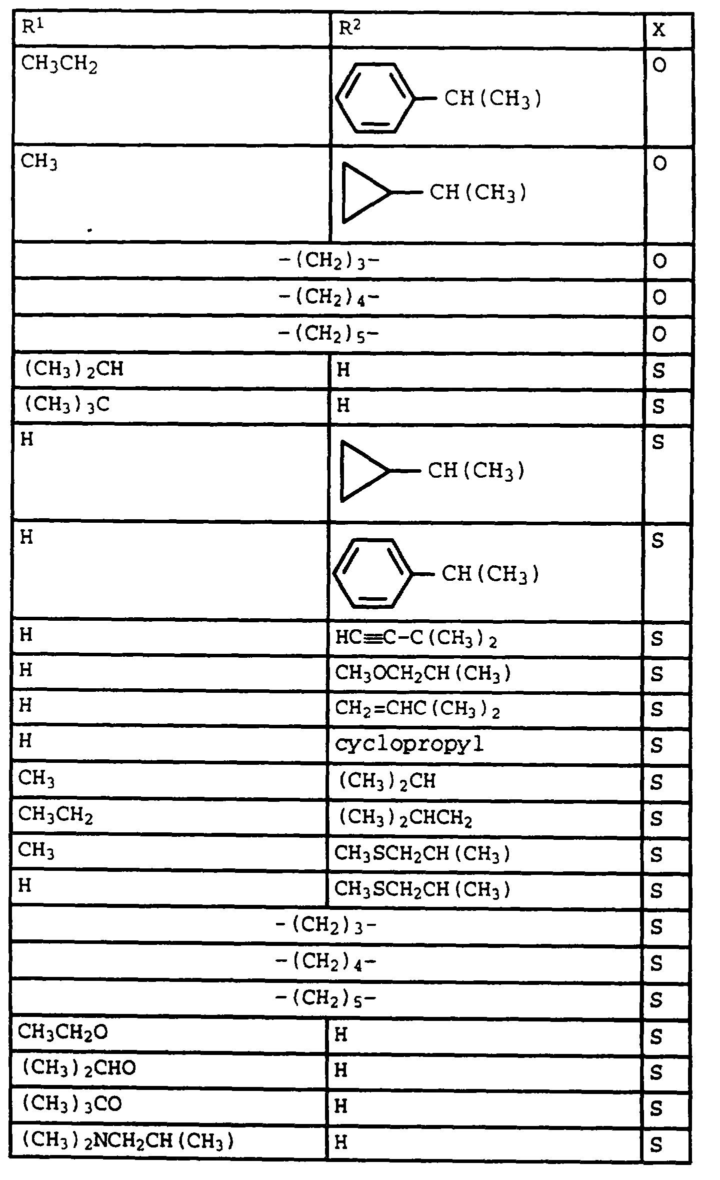

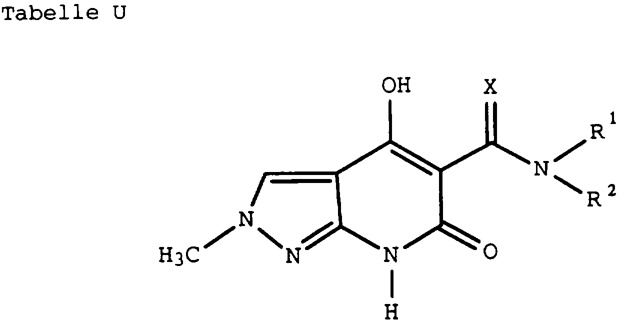

worin R¹, R und X die in Tabelle T genannte Bedeutung haben. 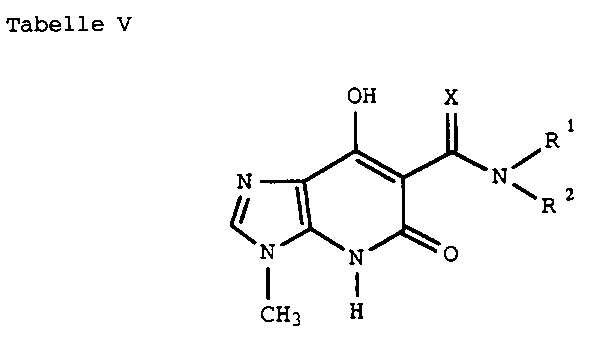 worin R¹, R und X die in Tabelle T im einzelnen genannte Bedeutung haben.

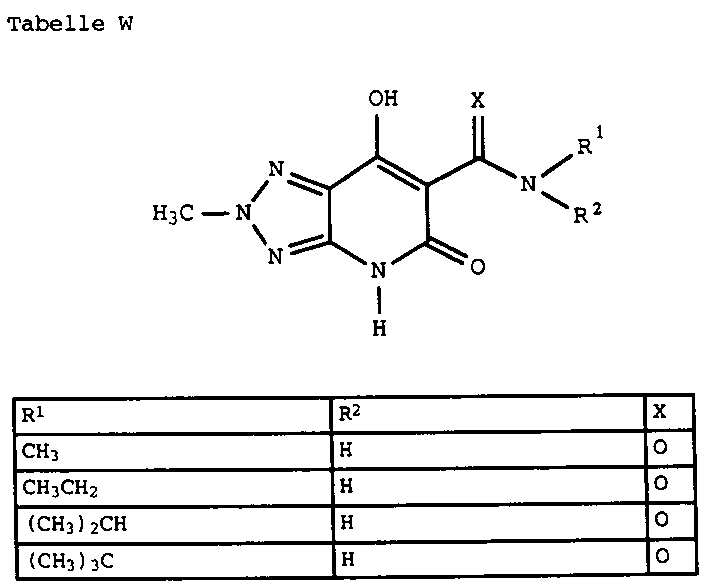

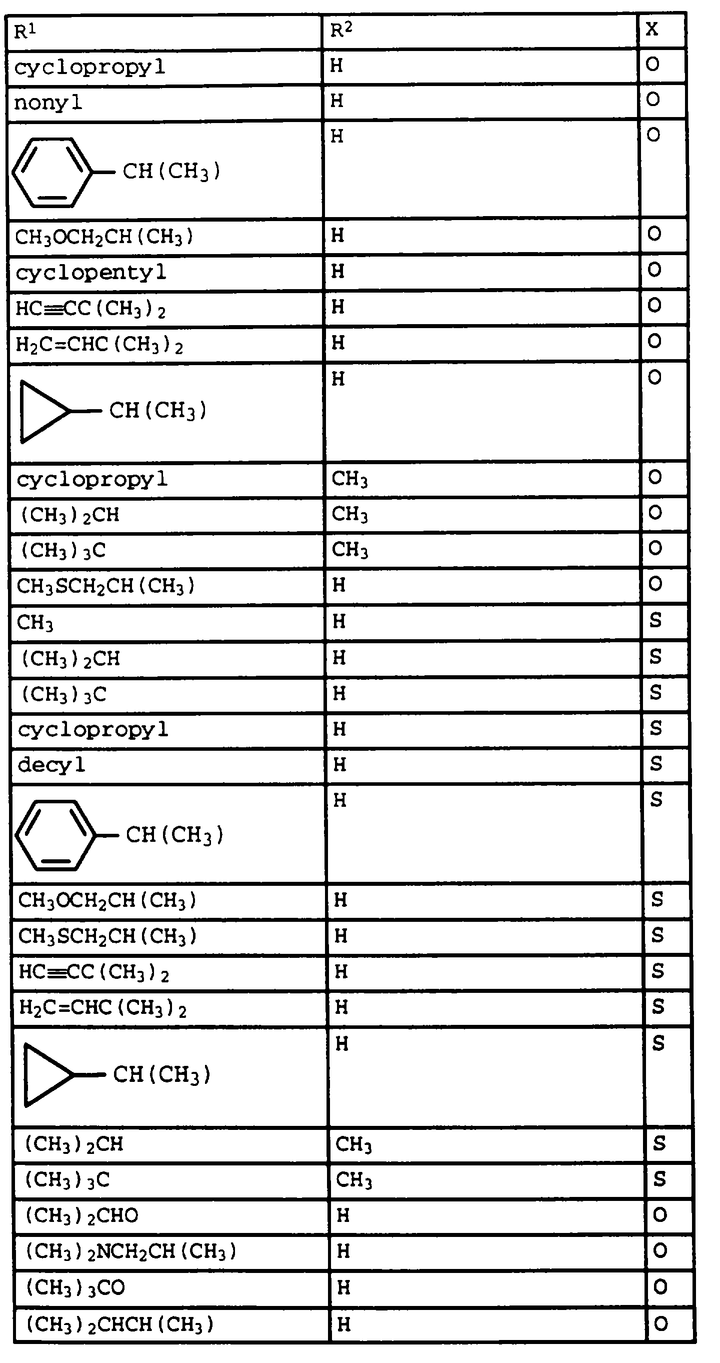

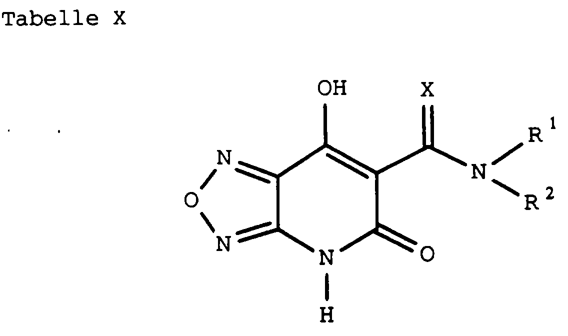

worin R¹, R und X die in Tabelle W im einzelnen genannte Bedeutung haben. 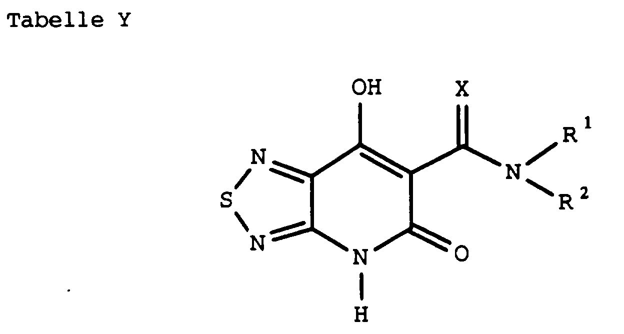 worin R¹, R und X die in Tabelle W im einzelnen genannte Bedeutung haben.

Die in den vorstehenden Tabellen speziell aufgeführten Heterocyclen Q sind unabhangig von der Bedeutung X, bzw. der Substituenten R¹ und R als besonders bevorzugt im Rahmen der allgemeinen Formel I anzusehen.

Die Verbindungen I bzw. die sie enthaltenden herbiziden Mittel können beispielsweise in Form von direkt versprühbaren Lösungen, Pulvern, Suspensionen, auch hochprozentigen wäßrigen, öligen oder sonstigen Suspensionen oder Dispersionen, Emulsionen, Öldispersionen, Pasten, Staubemitteln, Streumitteln oder Granulaten durch Versprühen, Vernebeln, Verstäuben, Verstreuen oder Gießen angewendet werden. Die Anwendungsformen richten sich nach den Verwendungszwecken; sie sollten in jedem Fall möglichst die feinste Verteilung der erfindungsgemäßen Wirkstoffe gewährleisten.

Die Verbindungen I eignen sich allgemein zur Herstellung von direkt versprühbaren Lösungen, Emulsionen, Pasten oder Öldispersionen. Als inerte Zusatzstoffe kommen Mineralölfraktionen von mittlerem bis hohem Siedepunkt, wie Kerosin oder Dieselöl, ferner Kohlenteeröle sowie Öle pflanzlichen oder tierischen Ursprungs, aliphatische, cyclische und aromatische Kohlenwasserstoffe, z.B. Toluol, Xylol, Paraffin, Tetrahydronaphthalin, alkylierte Naphthaline oder deren Derivate, Methanol, Ethanol, Propanol, Butanol, Cyclohexanol, Cyclohexanon, Chlorbenzol, Isophoron oder stark polare Lösungsmittel, wie N,N-Dimethylformamid, Dimethylsulfoxid, N-Methylpyrrolidon oder Wasser in Betracht.

Wäßrige Anwendungsformen können aus Emulsionskonzentraten, Dispersionen, Pasten, vernetzbaren Pulvern oder wasserdispergierbaren Granulaten durch Zusatz von Wasser bereitet werden. Zur Herstellung von Emulsionen, Pasten oder Öldispersionen können die Substrate als solche oder in einem Öl oder Lösungsmittel gelost, mittels Netz-, Haft-, Dispergier- oder Emulgiermittel in Wasser homogenisiert werden. Es können aber auch aus wirksamer Substanz, Netz-, Haft-, Dispergier- oder Emulgiermittel und eventuell Lösungsmittel oder Öl bestehende Konzentrate hergestellt werden, die zur Verdünnung mit Wasser geeignet sind.

Als oberflächenaktive Stoffe kommen die Alkali-, Erdalkali-, Ammoniumsalze von aromatischen Sulfonsäuren, z.B. Lignin-, Phenol-, Naphthalin- und Dibutylnaphthalinsulfonsäure, sowie von Fettsäuren, Alkyl- und Alkylarylsulfonaten, Alkyl-, Lauryl- ether- und Fettalkoholsulfaten, sowie Salze sulfatierter Hexa-, Hepta- und Octadecanolen, sowie von Fettalkoholglykolether, Kondensationsprodukte von sulfoniertem Naphthalin und seiner Derivate mit Formaldehyd, Kondensationsprodukte des Naphthalins bzw. der Naphthalinsulfonsäuren mit Phenol und Formaldehyd, Polyoxyethylenoctylphenolether, ethoxyliertes Isooctyl-, Octyl- oder Nonylphenol, Alkylphenol-, Tributylphenylpolyglykolether, Alkylarylpolyetheralkohole, Isotridecylalkohol, Fettalkoholethylenoxid-Kondensate, ethoxyliertes Rizinusöl, Polyoxyethylenalkylether oder Polyoxypropylen, Laurylalkoholpolyglykoletheracetat, Sorbitester, Lignin-Sulfitablaugen oder Methylcellulose in Betracht.

Pulver-, Streu- und Stäubemittel können durch Mischen oder gemeinsames Vermahlen der wirksamen Substanzen mit einem festen Trägerstoff hergestellt werden.

Granulate, z.B. Umhüllungs-, Inprägnierungs- und Homogengranulate können durch Bindung der Wirkstoffe an feste Tragerstoffe hergestellt werden. Feste Tragerstoffe sind Mineralerden wie Silicagel, Kieselsäuren, Kieselgele, Silikate, Talkum, Kaolin, Kalkstein, Kalk, Kreide, Bolus, Löß, Ton, Dolomit, Diatomeenerde, Calcium- und Magnesiumsulfat, Magnesiumoxid, gemahlene Kunststoffe, Düngemittel, wie Ammoniumsulfat, Ammoniumphosphat, Ammoniumnitrat, Harnstoffe und pflanzliche Produkte, wie Getreidemehl, Baumrinden-, Holz- und Nußschalenmehl, Cellulosepulver oder andere feste Trägerstoffe.

Die Formulierungen enthalten zwischen 0,1 und 95 Gew.-%, vorzugsweise zwischen 0,5 und 90 Gew.-%, Wirkstoff. Die Wirkstoffe werden dabei in einer Reinheit von 90 bis 100 %, vorzugsweise 95 bis 100 % (nach NMR-Spektrum) eingesetzt.

Die erfindungsgemäßen Verbindungen I können beispielsweise wie folgt formuliert werden:
I. Man vermischt 90 Gew.-Teilen der Verbindung Nr. 1.001 mit 10 Gew.-Teilen N-Methyl-α-pyrrolidon und erhalt eine Lösung, die zur Anwendung in Form kleinster Tropfen geeignet ist;
II. 20 Gew.-Teile der Verbindung Nr. 3.002 werden in einer Mischung gelöst, die aus 80 Gew.Teilen Xylol, 10 Gew.-Teilen des Anlagerungsproduktes von 8 bis 10 Mol Ethylenoxid an 1 Mol Ölsäure-N-monoethanolamid, 5 Gew.Teilen Calciumsalz der Dodecylbenzolsulfonsäure, 5 Gew.- Teilen des Anlagerungsproduktes von 40 Mol Ethylenoxid an 1 Mol Ricinusöl besteht. Durch Ausgießen und feines Verteilen der Lösung in 100.000 Gew.-Teilen Wasser erhält man eine wäßrige Dispersion, die 0,02 Gew.-% des Wirkstoffs enthält.
III. 20 Gew.-Teile der Verbindung Nr. 3.003 werden in einer Mischung gelöst, die aus 40 Gew.-Teilen Cyclohexanon, 30 Gew.-Teilen Isobutanol, 20 Gew.-Teilen des Anlagerungsproduktes von 7 Mol Ethylenoxid an 1 Mol Isooctylphenol und 10 Gew.-Teilen des Anlagerungsproduktes von 40 Mol Ethylenoxid an 1 Mol Ricinusöl besteht. Durch Eingießen und feines Verteilen der Lösung in 100.000 Gew.-Teilen Wasser erhalt man eine wäßrige Dispersion, die 0,02 Gew.-% des Wirkstoffs enthält.
IV. 20 Gew.-Teile des Wirkstoffs Nr. 3.012 werden in einer Mischung gelöst, die aus 25 Gew.-Teilen Cyclohexanon, 65 Gew.-Teilen einer Mineralölfraktion vom Siedepunkt 210 bis 280°C und 10 Gew.-Teilen des Anlagerungsproduktes von 40 Mol Ethylenoxid an 1 Mol Ricinusöl besteht. Durch Eingießen und feines Verteilen der Lösung in 100.000 Gew.-Teilen Wasser erhält man eine wäßrige Dispersion, die 0,02 Gew.-% des Wirkstoffs enthält.
V. 20 Gew.-Teile des Wirkstoffs Nr. 3.013 werden mit 3 Gew.-Teilen des Natriumsalzes der Diisobutylnaphtalin-α-sulfonsäure, 1 Gew.-Teilen des Natriumsalzes einer Ligninsulfonsäure aus einer Sulfitablauge und 60 Gew.-Teilen pulverförmigem Kieselsäuregel gut vermischt und in einer Hammermühle vermahlen. Durch feines Verteilen der Mischung in 20.000 Gew.-Teilen Wasser erhält man eine Spritzbrühe, die 0,1 Gew.-% des Wirkstoffs enthält.
VI. 3 Gew.-Teilen des Wirkstoffs Nr. 3.004 werden mit 97 Gew.-Teilen feinteiligem Kaolin vermischt. Man erhalt auf diese Weise ein Stäubemittel, das 3 Gew.-% des Wirkstoffs enthält.
VII. 30 Gew.-Teilen des Wirkstoffs Nr. 3.009 werden mit einer Mischung aus 92 Gew.-Teilen pulverförmigem Kieselsäuregel und 8 Gew.-Teilen Paraffinöl, das auf die Oberfläche dieses Kieselsäuregels gesprüht wurde, innig vermischt. Man erhalt auf diese Weise eine Aufbereitung des Wirkstoffs mit guter Haftfähigkeit.
VIII. 20 Gew.-Teile des Wirkstoffs Nr. 3.002 werden mit 2 Gew.-Teilen Calciumsalz der Dodecylbenzolsulfonsäure, 8 Gew.-Teilen Fettalkohol-polyglykolether, 2 Gew.-Teilen Natriumsalz eines Phenolsulfonsäure-harnstoff-formaldehyd-Kondensates und 68 Gew.-Teilen eines paraffinischen Mineralöls innig vermischt. Man erhält eine stabile ölige Dispersion.

Die Applikation der herbiziden Mittel bzw. der Wirkstoffe kann im Vorauflauf- oder im Nachauflaufverfahren erfolgen. Sind die Wirkstoffe für gewisse Kulturpflanzen verträglich, so können Ausbringungstechniken angewandt werden, bei welchen die herbiziden Mittel mit Hilfe der Spritzgeräte so gespritzt werden, daß die Blätter der empfindlichen Kulturpflanzen nach Möglichkeit nicht getroffen werden, während die Wirkstoffe auf die Blätter darunter wachsender unerwünschter Pflanzen oder die unbedeckte Bodenfläche gelangen (post-directed, lay-by).

Die Aufwandmengen an Wirkstoff betragen je nach Bekämpfungsziel, Jahreszeit, Zielpflanzen und Wachstumsstadium 0,001 bis 3, vorzugsweise 0,01 bis 1 kg/ha aktive Substanz (a.S.).

In Anbetracht der Vielseitigkeit der Applikationsmethoden können die erfindungsgemäßen Verbindungen bzw. sie enthaltende Mittel noch in einer weiteren Zahl von Kulturpflanzen zur Beseitigung unerwünschter Pflanzen eingesetzt werden. In Betracht kommen beispielsweise folgende Kulturen:

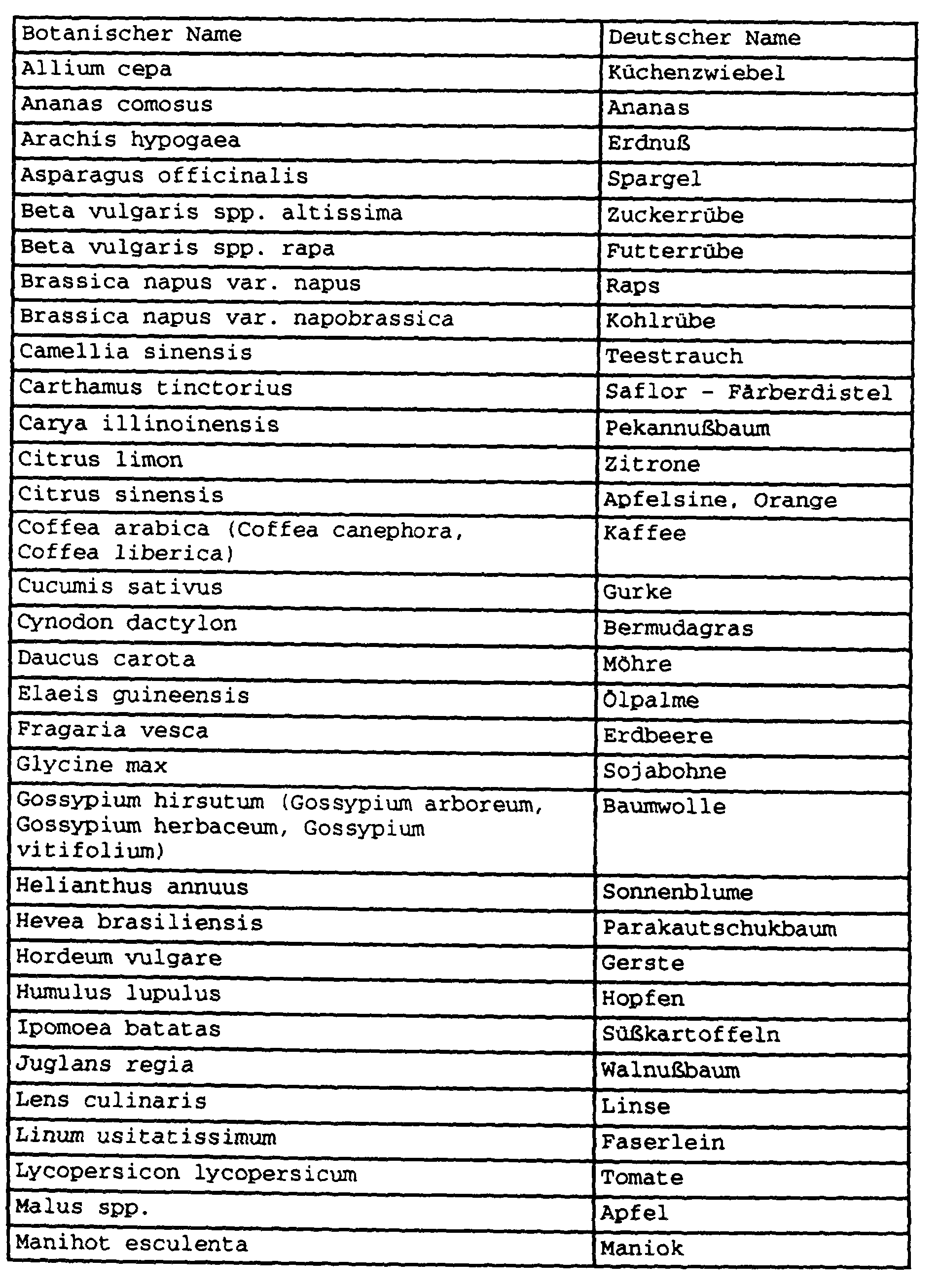

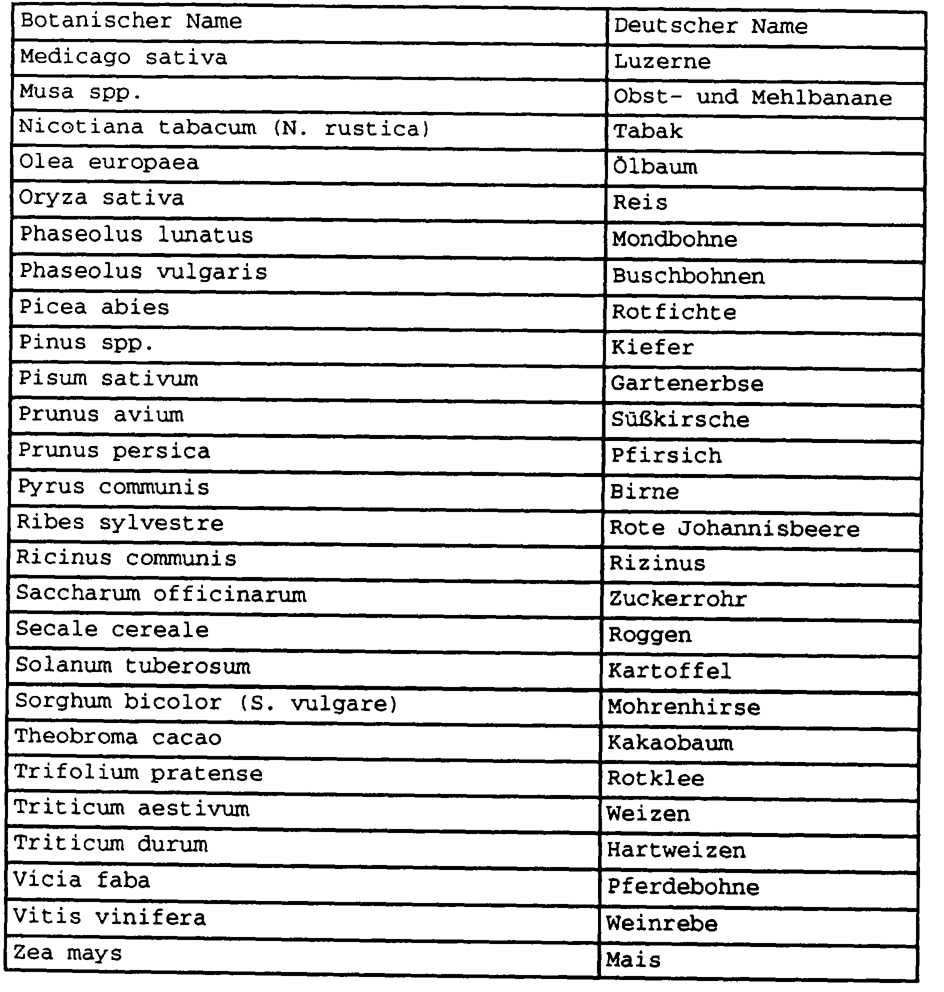

Zur Verbreiterung des Wirkungsspektrums und zur Erzielung synergistischer Effekte können die Hydroxypyridoncarbonsäureamide I mit zahlreichen Vertretern anderer herbizider oder wachstumsregulierender Wirkstoffgruppen gemischt und gemeinsam ausgebracht werden. Beispielsweise kommen als Mischungspartner Diazine, 4H-3,1-Benzoxazinderivate, Benzothiadiazinone, 2,6-Dinitroaniline, N-Phenylcarbamate, Thiolcarbamate, Halogencarbonsäuren, Triazine, Amide, Harnstoffe, Diphenylether, Triazinone, Uracile, Benzofuranderivate, Cyclohexan-1,3-dionderivate, Chinolincarbonsäurederivate, Sulfonylharnstoffe, Aryloxy-, Heteroaryloxyphenoxypropionsäuren sowie deren Salze, Ester und Amide und andere in Betracht.

Außerdem kann es von Nutzen sein, die neuen Verbindungen I allein oder in Kombination mit anderen Herbiziden auch noch mit weiteren Pflanzenschutzmitteln gemischt gemeinsam auszubringen, beispielsweise mit Mitteln zur Bekämpfung von Schädlingen oder phytopathogenen Pilzen bzw. Bakterien. Von Interesse ist ferner die Mischbarkeit mit Mineralsalzlösungen, welche zur Behebung von Ernährungs- und Spurenelementmängeln eingesetzt werden. Es können auch nichtphytotoxische Öle und Ölkonzentrate zugesetzt werden.

### Synthesebeispiele

### Beispiel 1

### 6-[(1,1-Dimethylethyl-)aminocarbonyl-]-7-hydroxy-thieno[3,2-b]-pyridin-5-(4H)-on (Nr. 3.002 in Tabelle 3)

3,1 g (13 mmol) 6-Ethoxycarbonyl-7-hydroxy-thieno[3,2-b]pyridin-5(4H)-on und 1,14 g (1,2 eq.) tert.-Butylamin in 80 ml EtOH wurden 8 h bei 150°C im Autoklaven gerührt. Das Lösungsmittel wurde entfernt und der feste Rückstand mit Diethylether/Aceton/ Ethanol (1/1/1) verrührt. Die Kristalle wurden abgesaugt, mit Diethylether gewaschen und getrocknet.

Ausbeute: 1,3 g (38 %), Fp 270 - 271°C

### Beispiel 2

### 6-[(1,1-Dimethylpropinyl-)aminocarbonyl-]-7-hydroxy-thieno[3,2-b] pyridin-5(4H)-on (Nr. 3.012 in Tabelle 3)

3,1 g (13 mmol) 6-Ethoxycarbonyl-7-hydroxy-thieno[3,2-b]pyridin-5(4H)-on und 1,3 g (1,2 eq. 1,1-Dimethylpropargylamin in 80 ml EtOH wurden 8 h bei 150°C im Autoklaven gerührt. Das Lösungsmittel wurde im Vakuum entfernt und der feste Rückstand mit Diethylether verrührt. Der Feststoff wurde abgesaugt und chromatographisch (Kieselgel, Methylenchlorid/Methanol = 8/1) gereinigt.

Ausbeute: 0,4 g (11 %), Fp 206 - 208°C

In entsprecher Weise wurden unter Abwandlung der Ausgangsstoffe weitere Verbindungen der Formel I hergestellt. Die so erhaltenen Verbindungen sind in den nachstehenden Tabellen mit physikalischen Daten aufgelistet.

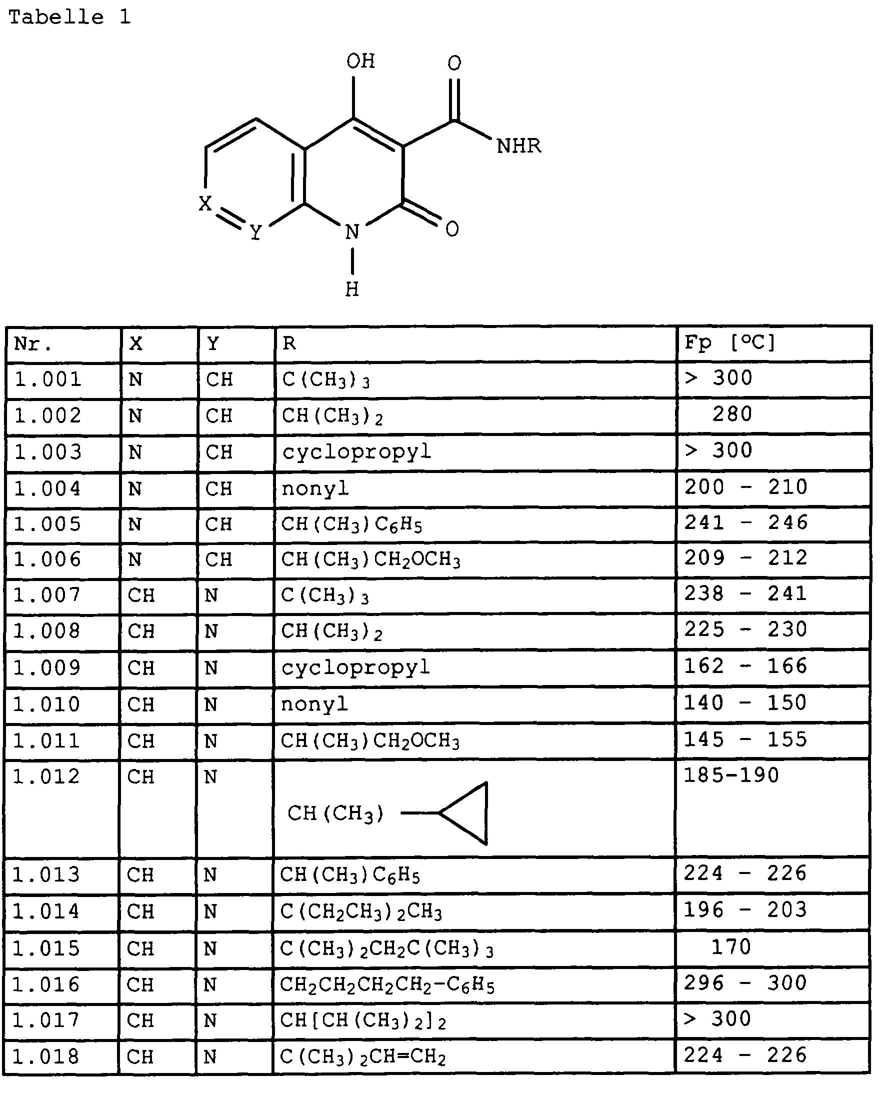

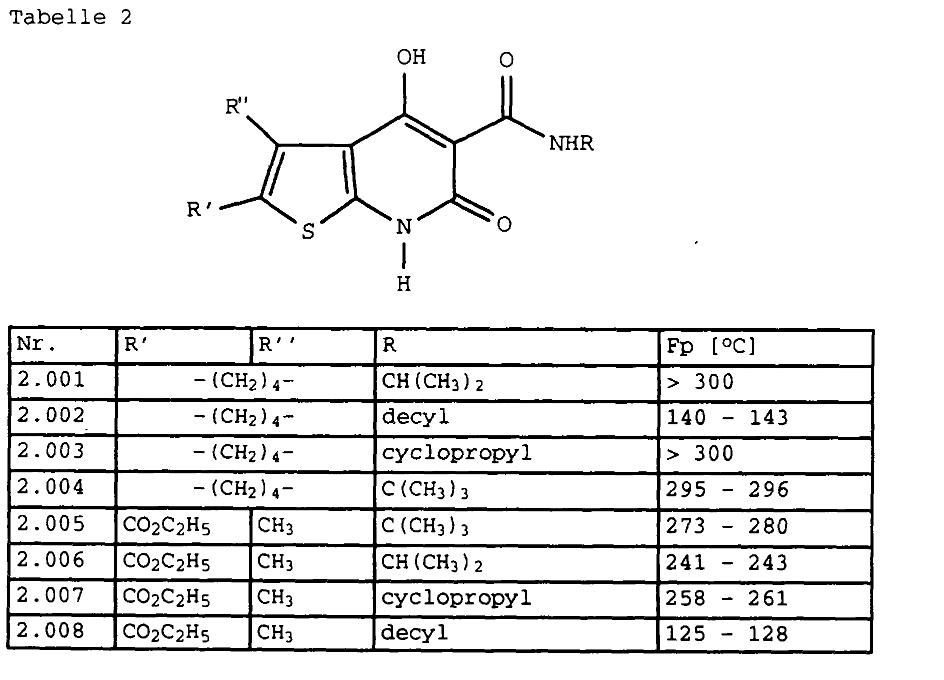

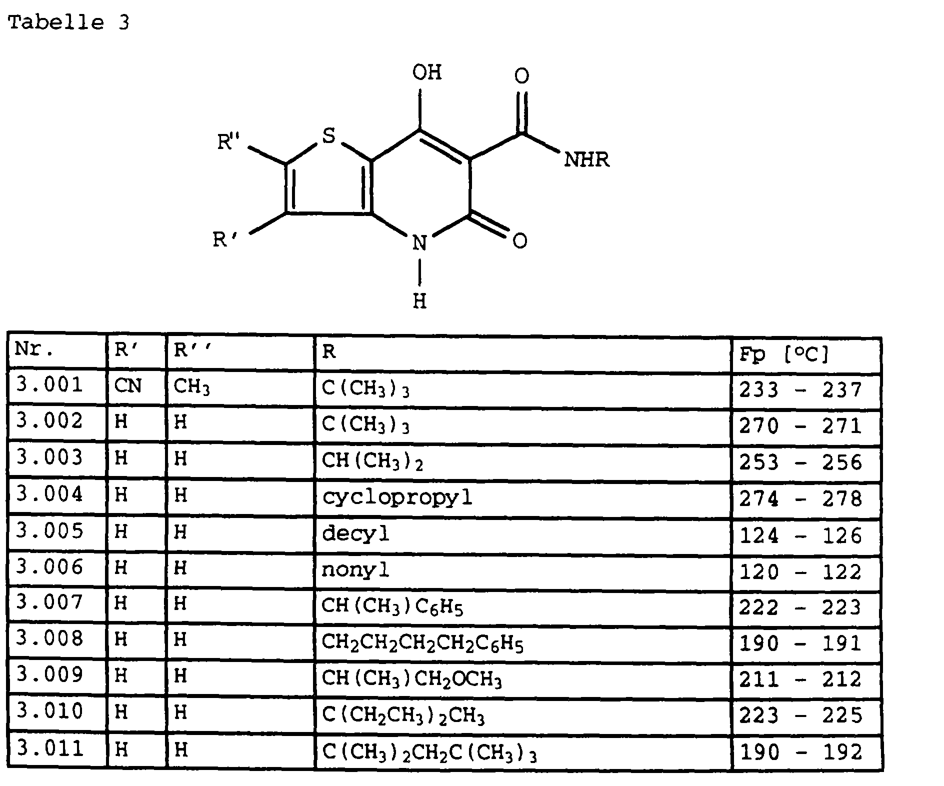

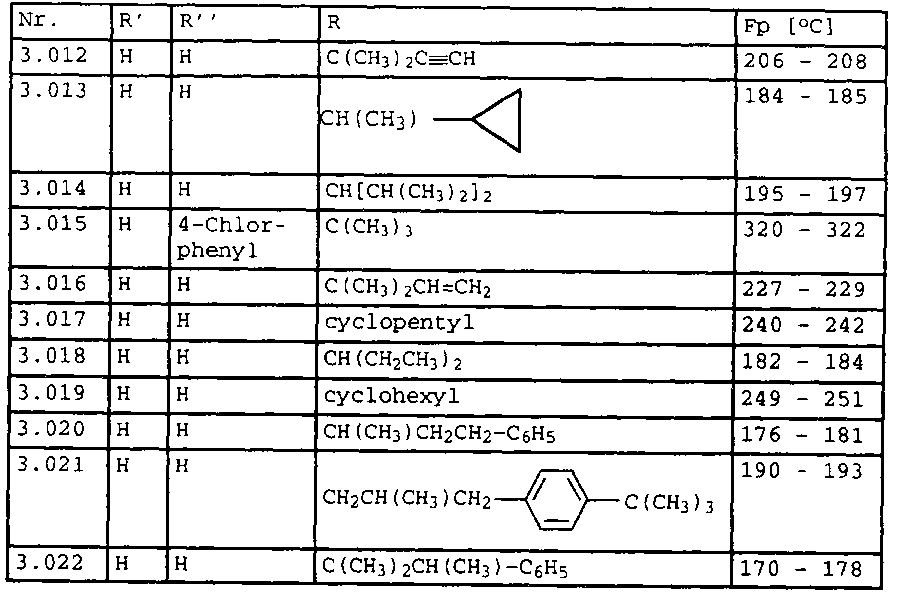

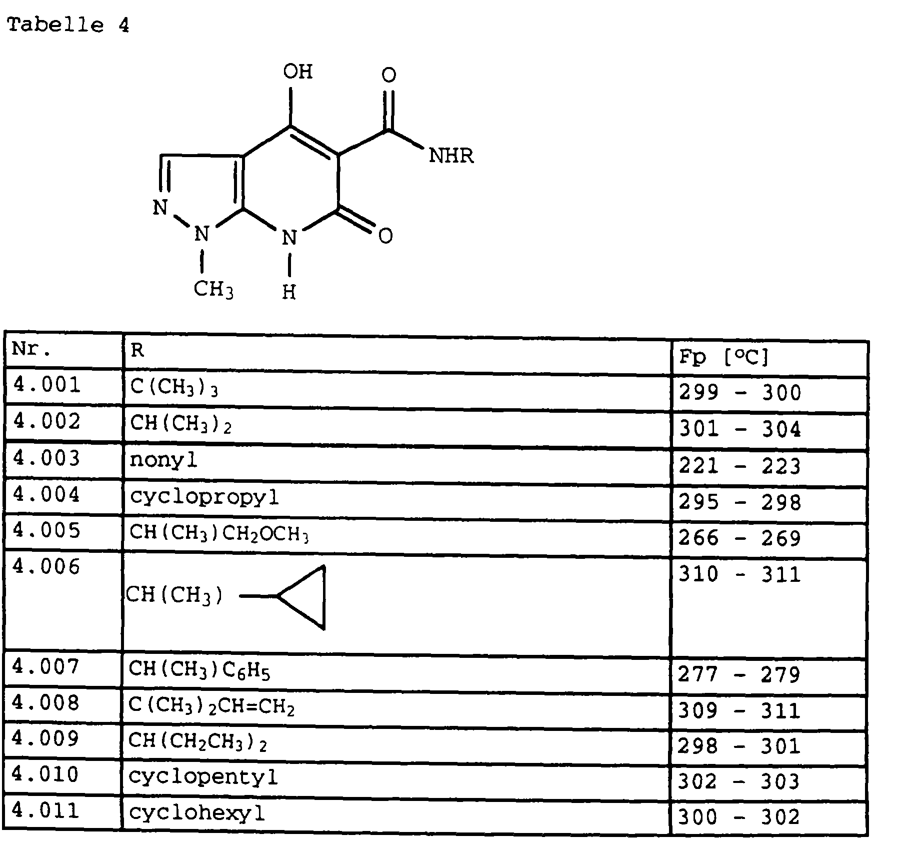

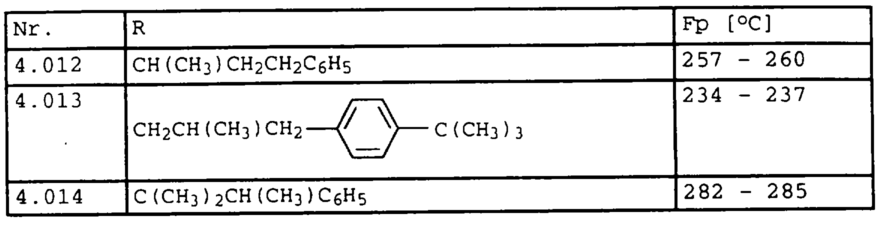

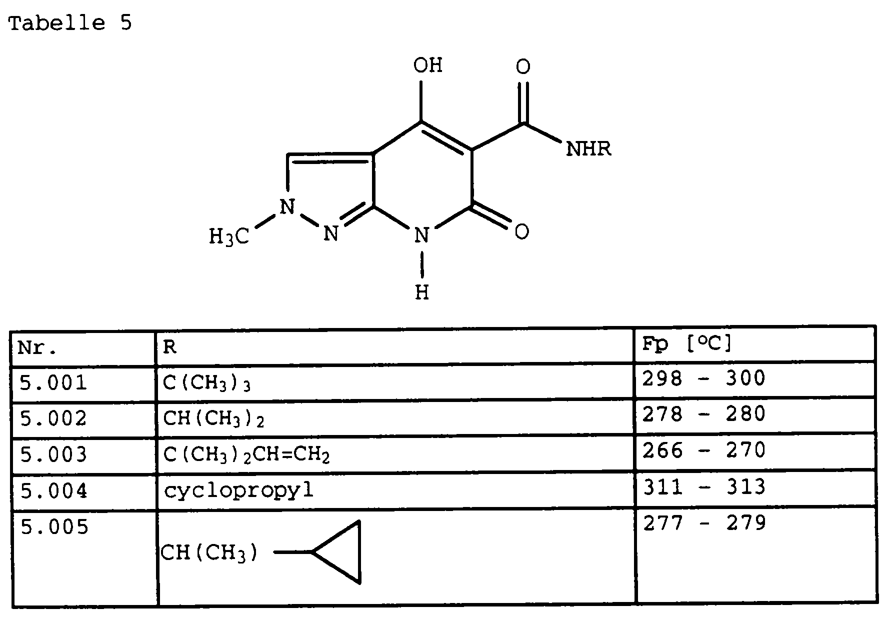

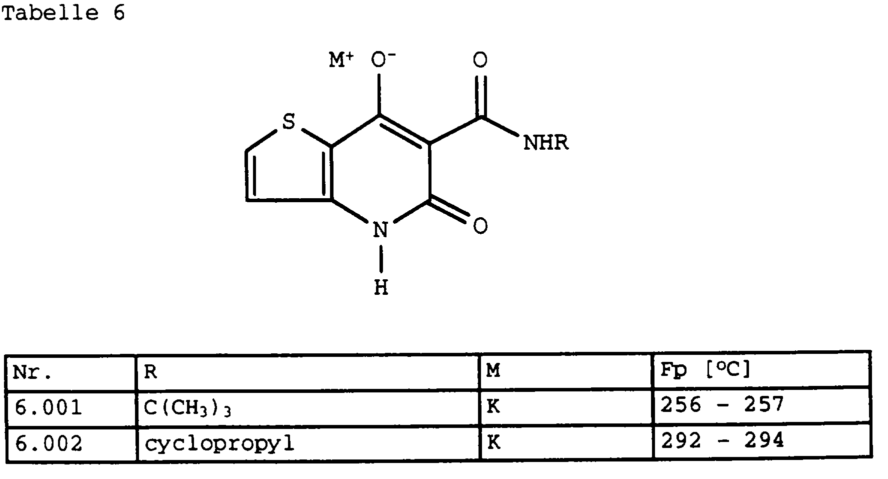

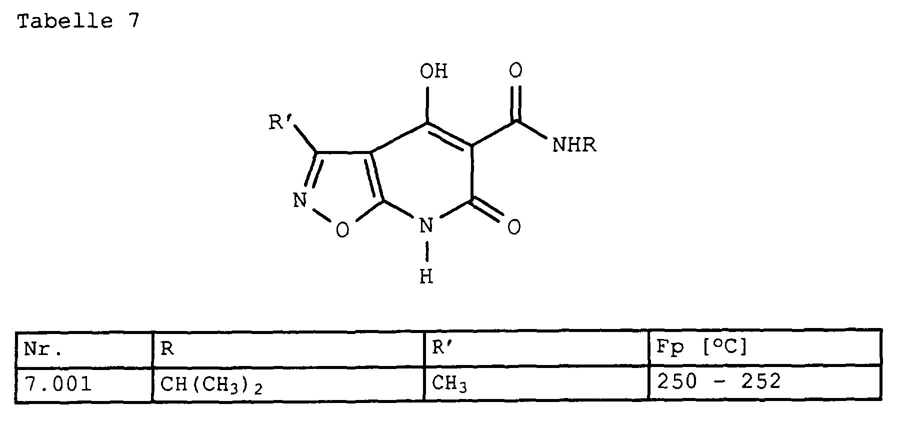

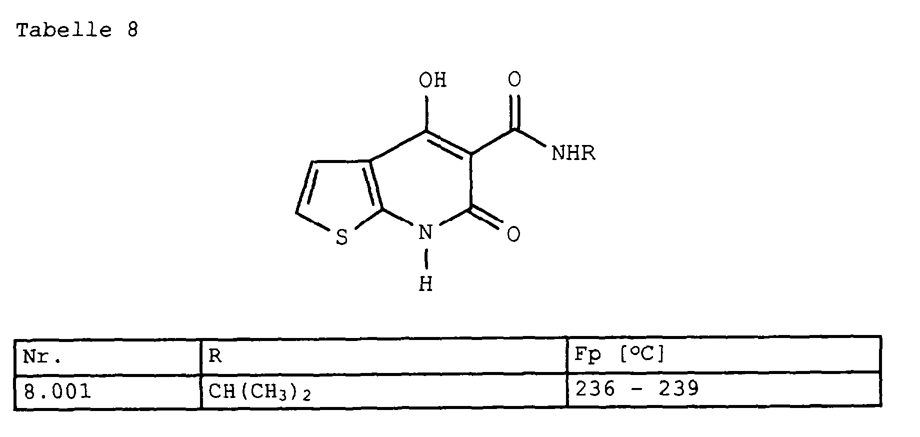

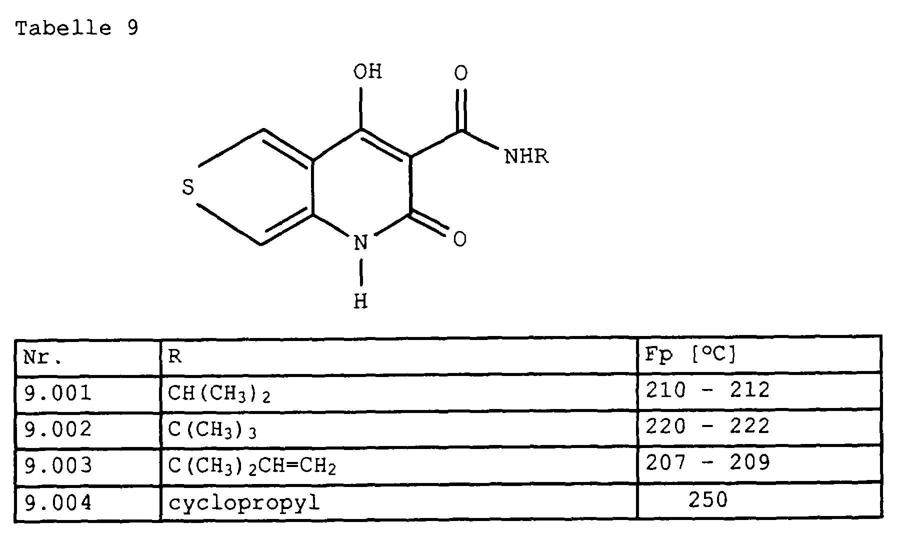

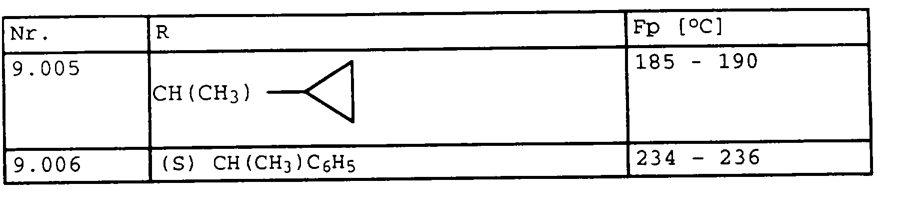

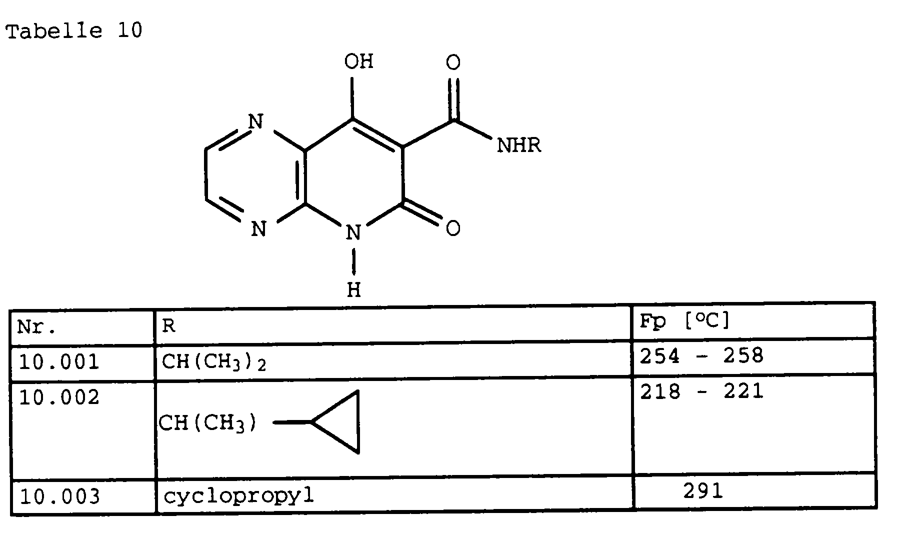

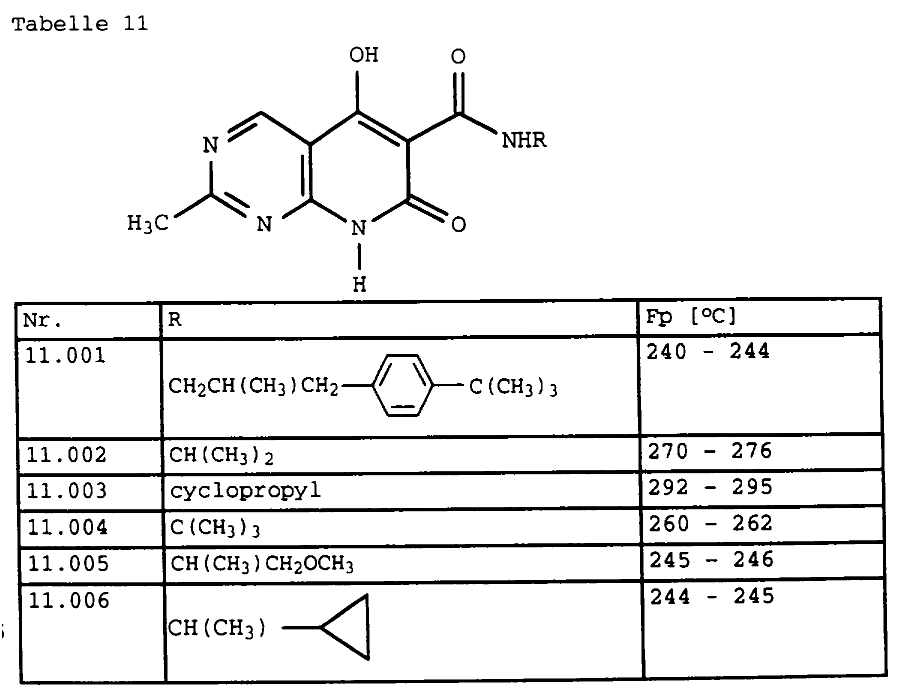

### Anwendungsbeispiele

Die herbizide Wirkung der Verbindungen I ließ sich durch Gewächshausversuche zeigen:

Als Kulturgefäße dienten Plastikblumentöpfe mit lehmigem Sand mit etwa 3,0 % Humus als Substrat. Die Samen der Testpflanzen wurden nach Arten getrennt eingesät.

Bei Vorauflaufbehandlung wurden die in Wasser suspendierten oder emulgierten Wirkstoffe direkt nach Einsaat mittels fein verteilender Düsen aufgebracht. Die Gefäße wurden leicht beregnet, um Keimung und Wachstum zu fördern und anschließend mit durchsichtigen Plastikhauben abgedeckt, bis die Pflanzen angewachsen waren. Diese Abdeckung bewirkt ein gleichmäßiges Keimen der Testpflanzen, sofern dies nicht durch die Wirkstoffe beeinträchtigt wurde.

Zum Zwecke der Nachauflaufbehandlung wurden die Testpflanzen je nach Wuchsform erst bei einer Wuchshöhe von 3 bis 15 cm mit den in Wasser suspendierten oder emulgierten Wirkstoffen behandelt. Die Aufwandmenge für die Nachauflaufbehandlung betrug 3 kg/ha a.S.

Die Pflanzen wurden artenspezifisch bei Temperaturen von 10-25°C bzw. 20-35°C gehalten. Die Versuchsperiode erstreckte sich über 2 bis 4 Wochen. Während dieser Zeit wurden die Pflanzen gepflegt und ihre Reaktion auf die einzelnen Behandlungen wurde ausgewertet.

Bewertet wurde nach einer Skala von 0 bis 100. Dabei bedeutet 100 kein Aufgang der Pflanzen bzw. völlige Zerstörung zumindest der oberirdischen Teile und 0 keine Schädigung oder normaler Wachstumsverlauf.

Die in den Gewächshausversuchen verwendeten Pflanzen setzten sich aus folgenden Arten zusammen:

| | |
|---|---|
| Lateinischer Name | Deutscher Name |
| Centaurea cyanus | Kornblume |
| Setaria italica | Ital. Raygras |
| Bromus spp. | Trespenarten |

Mit 3 kg/ha a.S. im Nachauflaufverfahren eingesetzt, erzielt man mit den Beispielen 3.002-3.004 und 3.009, 3.012 und 3.013 sehr gute Wirkung gegen die genannten unerwünschten Pflanzen. Beispiel 1.001 bekämpft wirksam Centaurea cyanus.

Verbindungen Nr. 3.007, 3.009 und 3.012 wurden mit einer Aufwandmenge von 1 kg/ha a.S. in den Kulturen Reis, Weizen, Soja und Baumwolle getestet. Dabei wurde eine gute herbizide Wirkung gegenüber Digitaria sanguinalis, Abutilon theophrasti, Amaranthus retroflexus, Chenopodium album und Matricaria inodora festgestellt.

## Patentansprüche

1. Heteroaromatisch kondensierte Hydroxypyridoncarbonsäureamide der Struktur I 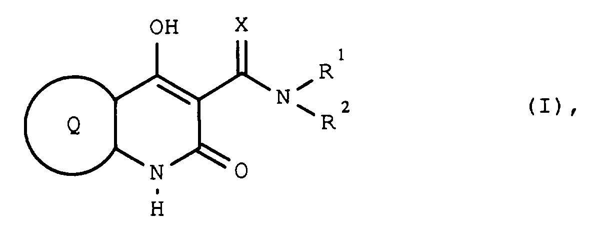 in der die Substituenten und der Ring Q folgende Bedeutung haben:
R¹ Wasserstoff, Hydroxyl, C₁-C₁₂-Alkyl, C₃-C₁₂-Alkenyl, C₃-C₁₂-Alkinyl, C₃-C₈-Cycloalkyl, C₁-C₁₂-Alkoxy, wobei diese Gruppen folgende Reste tragen können: Halogen, Cyano, C₃-C₈-Cycloalkyl, C₁-C₄-Alkoxy, C₁-C₄-Alkylthio, C₁-C₄-Halogenalkylthio, C₁-C₄-Halogenalkoxy, Di-C₁-C₄-Alkylamino, Phenyl, Phenoxy, Phenylthio, wobei die Phenylreste ihrerseits eine bis drei der folgenden Gruppen tragen können: Halogen, C₁-C₄-Alkyl, Cyano, Nitro, C₁-C₄-Alkoxy, C₁-C₄-Halogenalkyl, C₁-C₄-Halogenalkoxy;
R Wasserstoff, C₁-C₁₂-Alkyl, C₃-C₁₂-Alkenyl, C₃-C₁₂-Alkinyl, C₃-C₈-Cycloalkyl, C₃-C₈-Alkenyloxy, Di-C₁-C₄-Alkylamino, wobei diese Gruppen folgende Reste tragen können: Halogen, Cyano, C₃-C₈-Cycloalkyl, C₁-C₄-Alkoxy, C₁-C₄-Alkylthio, C₁-C₄-Halogenalkoxy, C₁-C₄-Halogenalkylthio, Phenyl, Phenoxy, Phenylthio, wobei die Phenylreste ihrerseits eine bis drei der folgenden Gruppen tragen können: Halogen,C₁-C₄-Alkyl, C₁-C₄-Alkoxy, Cyano, Nitro;
oder
R¹, R gemeinsam eine Alkylenkette mit 2 - 6 Gliedern bedeuten, welche durch Sauerstoff, Schwefel oder N-Methyl unterbrochen sein kann;
X Sauerstoff oder Schwefel;
Q ein fünf- oder sechsgliedriger heteroaromatischer Ring enthaltend 1 - 3 Stickstoffatome und/oder ein Sauerstoff- oder Schwefelatom als Heteroatome, welcher ein- bis drei der folgenden Reste tragen kann: Halogen, Nitro, Cyano, C₁-C₄-Alkyl, C₁-C₄-Alkoxy, C₁-C₄-Alkylthio, C₁-C₄-Alkoxy-C₁-C₄-alkyl, C₃-C₈-Cycloalkyl, C₂-C₄-Alkenyl, Phenyl, Phenyl-C₁-C₄-alkyl, wobei die Phenylreste ihrerseits ein bis drei der folgenden Gruppen tragen können: Halogen, C₁-C₄-Alkyl, C₁-C₄-Alkoxy, C₁-C₄-Alkylthio, C₁-C₄-Halogenalkyl, C₁-C₄-Halogenalkoxy;
und deren umweltverträglichen Salze.

2. Heteroaromatisch kondensierte Hydroxypyridoncarbonsäureamide der Formel I gemäß Anspruch 1, in der
R¹ Wasserstoff und R verzweigtes oder unverzweigtes C₁-C₁₂-Alkyl, C₃-C₁₂-Alkenyl, C₃-C₁₂-Alkinyl, C₁-C₁₂-Alkoxy, C₃-C₆-Cycloalkyl, wobei diese Gruppen folgende Reste tragen können: Halogen, C₁-C₄-Alkoxy, C₃-C₈-Cycloalkyl, gegebenenfalls durch Halogen oder C₁-C₄-Alkyl substituiertes Phenyl
bedeuten und Q und X die in Anspruch 1 gegebene Bedeutung haben.

3. Verfahren zur Herstellung der heteroaromatisch kondensierten Hydroxypyridoncarbonsäureamide der Formel I gemaß Anspruch 1, dadurch gekennzeichnet, daß man eine Carbonsäure bzw. ein Carbonsäurederivat der Formel II 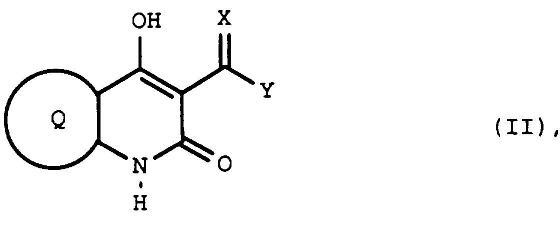 in der X und Q die in Anspruch 1 gegebene Bedeutung haben und Y Hydroxy, Halogen oder eine übliche nukleofuge Abgangsgruppe bedeutet, in an sich bekannter Weise mit einem substituierten Amin der Formel III 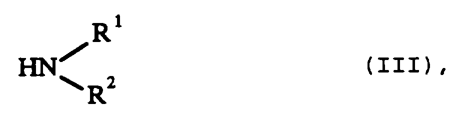 in der die Reste R¹ und R die in Anspruch 1 gegebene Bedeutung haben, umsetzt.

4. Herbizide Mittel, enthaltend ein heteroaromatisch kondensiertes Hydroxypyridoncarbonsäureamid der Formel I gemäß Anspruch 1 und inerte Zusatzstoffe.

5. Verfahren zur Bekämpfung unerwünschten Pflanzenwuchses, dadurch gekennzeichnet, daß man die unerwünschten Pflanzen und/oder ihren Lebensraum mit einer herbizid wirksamen Menge eines heteroaromatisch kondensierten Hydroxypyridoncarbonsaureamids der Formel I gemäß Anspruch 1 oder dessen umweltverträglichen Salzes behandelt.

## Claims

1. A heteroaromatically condensed hydroxypyridonecarboxamide of the structure I 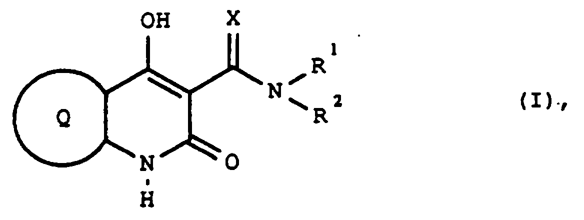 where the substituents and the ring Q have the following meanings:
R¹ is hydrogen, hydroxyl, C₁-C₁₂-alkyl, C₃-C₁₂-alkenyl, C₃-C₁₂-alkynyl, C₃-C₈-cycloalkyl or C₁-C₁₂-alkoxy, it being possible for these groups to carry the following radicals: halogen, cyano, C₃-C₈-cycloalkyl, C₁-C₄-alkoxy, C₁-C₄-alkylthio, C₁-C₄-haloalkylthio, C₁-C₄-haloalkoxy, di-C₁-C₄-alkylamino, phenyl, phenoxy or phenylthio, it being possible for the phenyl radicals in turn to carry from one to three of the following groups: halogen, C₁-C₄-alkyl, cyano, nitro, C₁-C₄-alkoxy, C₁-C₄-haloalkyl or C₁-C₄-haloalkoxy;
R is hydrogen, C₁-C₁₂-alkyl, C₃-C₁₂-alkenyl, C₃-C₁₂-alkynyl, C₃-C₈-cycloalkyl, C₃-C₈-alkenyloxy or di-C₁-C₄-alkylamino, it being possible for these groups to carry the following radicals: halogen, cyano, C₃-C₈-cycloalkyl, C₁-C₄-alkoxy, C₁-C₄-alkylthio, C₁-C₄-haloalkoxy, C₁-C₄-haloalkyothio, phenyl, phenoxy or phenylthio, it being possible for the phenyl radicals in turn to carry from one to three of the following groups: halogen, C₁-C₄-alkyl, C₁-C₄-alkoxy, cyano or nitro;
or
R¹ and R together denote an alkylene chain which has 2 - 6 members and may be interrupted by oxygen, sulfur or N-methyl;
X is oxygen or sulfur;
Q is a five- or six-membered heteroaromatic ring which contains 1 - 3 nitrogen atoms and/or one oxygen or sulfur atom as heteroatoms and may carry from one to three of the following radicals: halogen, nitro, cyano, C₁-C₄-alkyl, C₁-C₄-alkoxy, C₁-C₄-alkylthio, C₁-C₄-alkoxy-C₁-C₄-alkyl, C₃-C₈-cycloalky-, C₂-C₄-alkenyl, phenyl or phenyl-C₁-C₄-alkyl, it being possible for the phenyl radicals in turn to carry from one to three of the following groups: halogen, C₁-C₄-alkyl, C₁-C₄-alkoxy, C₁-C₄-alkylthio, C₁-C₄-haloalkyl or C₁-C₄-haloalkoxy;
or an environmentally tolerated salt thereof.

2. A heteroaromatically condensed hydroxypyridonecarboxamide of the formula I as claimed in claim 1, where
R¹ is hydrogen and R is branched or straight-chain C₁-C₁₂-alkyl, C₃-C₁₂-alkenyl, C₃-C₁₂-alkynyl, C₁-C₁₂-alkoxy or C₃-C₆-cycloalkyl, it being possible for these groups to carry the following radicals: halogen, C₁-C₄-alkoxy, C₃-C₈-cycloalkyl or unsubstituted or halogen- or C₁-C₄-alkyl-substituted phenyl,
and Q and X have the meanings given in claim 1.

3. A process for the preparation of a heteroaromatically condensed hydroxypyridonecarboxamide of the formula I as claimed in claim 1, wherein a carboxylic acid or a carboxylic acid derivative of the formula II 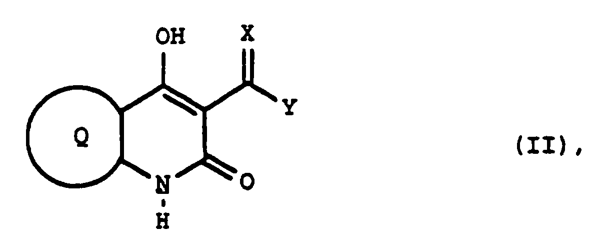 where X and Q have the meanings given in claim 1 and Y is hydroxyl, halogen or a customary nucleofugic leaving group, is reacted in a conventional manner with a substituted amine of the formula III 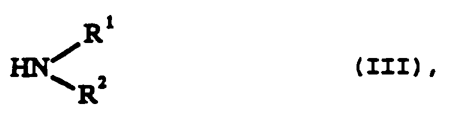 where R¹ and R have the meanings given in claim 1.

4. A herbicide containing a heteroaromatically condensed hydroxypyridonecarboxamide of the formula I as claimed in claim 1 and inert additives.

5. A method of controlling undesirable plant growth, wherein the undesirable plants and/or their habitat are treated with a herbicidal amount of a heteroaromatically condensed hydroxypyridonecarboxamide of the formula I as claimed in claim 1 or an environmentally tolerated salt thereof.

## Revendications

1. Hydroxypyridonecarboxamides hétéroaromatiques condensés de structure I 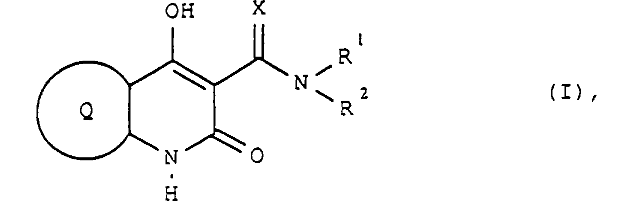 dans laquelle les symboles ont les significations suivantes :
R¹ représente l'hydrogène, un groupe hydroxy, alkyle en C1-C12, alcényle en C3-C12, alcynyle en C3-C12, cycloalkyle en C3-C8, alcoxy en C1-C12, ces groupes pouvant porter les substituants suivants : des halogènes, des groupes cyano, cycloalkyle en C3-C8, alcoxy en C1-C4, alkylthio en C1-C4, halogénoalkythio en C1-C4, halogénoalcoxy en C1-C4, di-(alkyle en C1-C4)-amino, phényle, phénoxy, phénylthio, les groupes phényle pouvant eux-mêmes porter un à trois des substituants suivants : les halogènes, les groupes alkyle en C1-C4, cyano, nitro, alcoxy en C1-C4, halogénoalkyle en C1-C4, halogénoalcoxy en C1-C4 ;
R représente l'hydrogène, un groupe alkyle en C1-C12, alcényle en C3-C12, alcynyle en C3-C12, cycloalkyle en C3-C8, alcényloxy en C3-C8, di-(alkyle en C1-C4)amino, ces groupes pouvant porter les substituants suivants : les halogènes, les groupes cyano, cycloalkyle en C3-C8, alcoxy en C1-C4, alkylthio en C1-C4, halogénoalcoxy en C1-C4, halogénoalkylthio en C1-C4, phényle, phénoxy, phénylthio, les groupes phényle pouvant eux-mêmes porter un à trois des substituants suivants : les halogènes, les groupes alkyle en C1-C4, alcoxy en C1-C4, cyano, nitro ;
ou bien
R¹ et R forment ensemble une chaîne alkylène de deux à six chaînons qui peut être interrompue par l'oxygène, le soufre ou un groupe N-méthyle ;
X représente l'oxygène ou le soufre ;
Q représente un cycle hétéroaromatique à cinq ou six chaînons contenant un à trois atomes d'azote et/ou un atome d'oxygène ou de soufre en tant qu'hétéroatomes, et qui peut porter un à trois des substituants suivants : les halogènes, les groupes nitro, cyano, alkyle en C1-C4, alcoxy en C1-C4, alkylthio en C1-C4, (alcoxy en C1-C4)alkyle en C1-C4, cycloalkyle en C3-C8, alcényle en C2-C4, phényle, phényl-alkyle en C1-C4, les groupes phényle pouvant eux-mêmes porter un à trois des substituants suivants : les halogènes, les groupes alkyle en C1-C4, alcoxy en C1-C4, alkylthio en C1-C4, halogénoalkyle en C1-C4, halogénoalcoxy en C1-C4 ;
et leurs sels non polluants.

2. Hydroxypyridonecarboxamides hétéroaromatiques condensés de formule I selon la revendication 1, pour lesquels
R¹ représente l'hydrogène et R un groupe alkyle en C1-C12, alcényle en C3-C12, alcynyle en C3-C12, alcoxy en C1-C12 à chaîne droite ou ramifiée, cycloalkyle en C3-C6, ces groupes pouvant eux-mêmes porter les substituants suivants : des halogènes, des groupes alcoxy en C1-C4, cycloalkyle en C3-C8, phényle eux-mêmes substitués éventuellement par des halogènes ou des groupes alkyle en C1-C4,
et Q et X ont les significations indiquées dans la revendication 1.

3. Procédé de préparation des hydroxypyridonecarboxamides hétéroaromatiques condensés de formule I de la revendication 1, caractérisé en ce que l'on fait réagir un acide carboxylique ou son dérivé de formule II 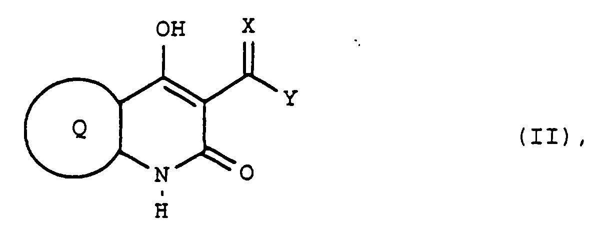 dans laquelle X et Q ont les significations indiquées dans la revendication 1 et Y représente un groupe hydroxy, un halogène ou un groupe éliminable nucléofuge usuel, de manière connue en soi, avec une amine substituée de formule III 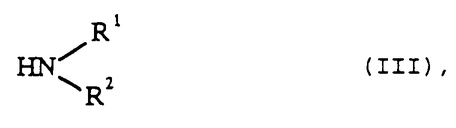 dans laquelle les symboles R¹ et R ont les significations indiquées dans la revendication 1.

4. Produits herbicides contenant un hydroxypyridonecarboxamide hétéroaromatique condensé de formule I de la revendication 1 et des additifs inertes.

5. Procédé pour combattre les croissances de végétaux indésirables, caractérisé en ce que l'on traite les végétaux indésirables et/ou leur habitat par une quantité herbicide efficace d'un hydroxypyridonecarboxamide hétéroaromatique condensé de formule I selon la revendication 1 ou de l'un de ses sels non polluants.
